# EUROPÄISCHE PATENTSCHRIFT

(11) **EP 0 646 595 B1**
(45) Veröffentlichungstag und Bekanntmachung des Hinweises auf die Patenterteilung: **04.11.1998**
(21) Anmeldenummer: 94113569.1
(22) Anmeldetag: 31.08.1994
(51) Int. Cl.: C07K 5/00, A61K 38/10

(54) **Nukleinsäuren-bindende Oligomere mit N-Verzweigung für Therapie und Diagnostik**
Nucleic acid binding oligomers having C-branches for therapy and diagnosis
Oligomères liant les acides nucléiques avec branchement en C et leur utilisation en thérapie et diagnostic

(30) Priorität: 13.09.1993 DE 4331012
(43) Veröffentlichungstag der Anmeldung: 05.04.1995
(73) Patentinhaber: BAYER AG, 51368 Leverkusen (DE)
(72) Erfinder: Löbberding, Dr. Antonius, D-42113 Wuppertal (DE); Mielke, Dr. Burkhard, D-51375 Leverkussen (DE); Schwemler,Christoph, D-42799 Leichlingen (DE); Schwenner, Dr. Eckhard, D-42113 Wuppertal (DE); Stropp, Dr. Udo, D-42781 Haan (DE); Springer, Dr. Wolfgang, D-42113 Wuppertal (DE); Kretschmer, Dr. Axel, D-51429 Bergisch Gladbach (DE); Pötter, Dr. Thorsten, D-51061 Köln (DE)

(56) Entgegenhaltungen:
- EP-A- 0 300 796
- CHEMICAL ABSTRACTS, Band 113, Nr. 21, 19. November 1990, Columbus, Ohio, USA P. WESTERMANN et al. "Preparation of oligodeoxy- ribonucleotide-based affinity carriers" Seite 776, Nr. 191 856t; & DD-A-273 444
- CHEMICAL ABSTRACTS, Band 94, Nr. 5, 2. Februar 1981, Columbus, Ohio, USA S.A. STREL'TSOV et al. "Specific interaction between oligovaline and nucleic acids" Seite 185, Nr. 26 354j; & Biofizika 1980, 25(5), 929-41

## Beschreibung

Das gezielte Abschalten von Genexpression durch komplementäre Nukleinsäuren, sogenannte Antisense-Oligonukleotide, stellt einen neuen Therapieansatz dar. Mögliche Anwendungen reichen von der Behandlung viraler Infektionen bis zur Therapie von Krebs (S. Agrawal, Tibtech 10, 152 (1992); W. James, Antiviral Chemistry & Chemotherapie 2, 191 (1991); B. Calabretta, Cancer Research 51, 4504 (1991)). Die Kontrolle der Genexpression erfolgt auf der Ebene von DNA und RNA und gelingt bereits mit unmodifizierten Oligonukleotiden (C. Helene, Anti-Cancer Drug Design 6, 569 (1991); E. Uhlmann, A. Peymann, Chemical Reviews 90, 543 (1990)). Diese sind jedoch aufgrund mangelnder enzymatischer Stabilität und zu geringer Aufnahme in zelluläre Systeme für therapeutische Anwendungen nicht geeignet. Therapeutische Anwendungen erfordern chemisch modifizierte Antisense-Oligonukleotide.

Oligonukleotide mit modifiziertem Internukleotidphosphat oder einer phosphatfreien Internukleotidverknüpfung wurden in vielen Arbeiten systematisch untersucht; ihre Synthese erwies sich jedoch als sehr aufwendig und beobachtete therapeutische Effekte als nicht ausreichend (E. Uhlmann, A. Peyman, Chemical Reviews 90, 543 (1990)).

Eine Alternative zur Modifikation oder Substitution der Phosphatgruppe in Nukleinsäuren ist der komplette Austausch von Ribose und Phosphat durch andere Rückgrate. Dieses Konzept wurde erstmals von Pitha et al. realisiert, der Ribosephosphat durch Poly-N-Vinyl-derivate ersetzte, was zu sogenannter "Plastik-DNA" führt (J. Pitha, P.O.P. Ts'O, J. Org. Chem. 33, 1341 (1968); J. Pitha, J. Adv. Polym. Sci. 50, 1 (1983)). Es erlaubt jedoch nicht den gezielten Aufbau definierter Sequenzen.

Die Synthese definierter Sequenzen gelingt, wenn anstelle von Zuckerphosphat beispielsweise ein Polyamid-Rückgrat verwendet wird, das in Analogie zur konventionellen Peptidsynthese (M. Bodanszky, Principles of Peptide Synthesis, Springer, Berlin 1984) schrittweise aufgebaut wird. Dieses Konzept wurde von verschiedenen Arbeitsgruppen unterschiedlich realisiert (J.E. Summerton et al. WO 86/05518; R. S. Varma et al. WO 92/18518; O. Buchardt et al. WO 92/20702; H. Wang, D. D. Weller, Tetrahedron Letters 32, 7385 (1991); P. Garner, J. U. Yoo, Tetrahedron Letters 34; 1275 (1993); S.-B. Huang, J. S. Nelson D. D. Weller; J. Org. Chem. 56; 6007 (1991)).

Polyamid-Nukleinsäuren eignen sich ebenfalls für diagnostische und molekular-biologische Anwendungen (Buchardt et al. WO 92/20703).

Bei der Bearbeitung derartiger Strukturen gelang die Synthese neuer N-verzweigter oligomerer Nukleinsäuren. Für diese wurde eine überraschend gute Bindung an DNA und RNA gefunden. Die Substanzen eignen sich zur Kontrolle von Genexpression und zeigen antivirale Eigenschaften. Weiterhin lassen sich derartige Substanzen in Diagnostik und Molekularbiologie zur Isolierung, Identifizierung und Quantifizierung von Nukleinsäuren verwenden.

Es wurden die nachfolgend beschriebenen Strukturen synthetisiert:

Die Erfindung betrifft Verbindungen der allgemeinen Formel (I), 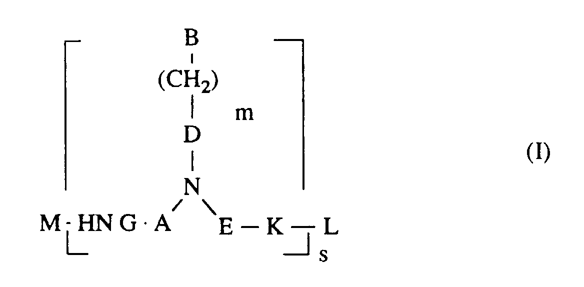 in der
- A: für -(CH₂)ₙ- oder -CO- steht,
- B: für alle natürlichen oder unnatürlichen Nukleobasen, wie z.B. Thymin, Uracil, Cytosin, Adenin, Guanin, Hypoxanthin, oder durch chemische Modifikation von diesen abgeleitete Derivate oder halogenierte Vorstufen von diesen, gegebenenfalls an den Aminogruppen mit Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert oder mit freien Aminogruppen steht,
- D: -(CO)ₚ- steht,
- E und G: unabhängig voneinander für -CHR- stehen, wobei
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-α-aminobuttersäure, anderen unnatürlichen Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Iodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, in ihrer D- oder L-Form oder gegebenenenfalls
- E und G: über eine Alkylkette -(CH₂)_{q}- miteinander verknüpft sind,
- K: für -CO-, -SO₂- oder -CH₂- steht,
- L: ein Carrier-System, Reporter-Ligand, eine löslichkeitsvermittelnde Gruppe oder Wasserstoff sein kann,
- M: unabhängig von L ein Carrier-System, Reporter-Ligand, eine löslichkeitsvermittelnde Gruppe oder Wasserstoff sein kann,
- m: 0, 1, 2 oder 3 sein kann,
- n: 0, 1, 2, 3 oder 4 sein kann,
- p: 0, 1 oder 2 sein kann,
- q: 0, 1 oder 2 sein kann, und
- s: Werte zwischen 1 und 30 annehmen kann.

Bevorzugt sind Verbindungen der allgemeinen Formel (I),
in der
- A: für -(CH₂)ₙ- oder -CO- steht,
- B: für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
- D: für -(CO)ₚ- steht,
- E und G: unabhängig voneinander für -CHR- stehen, wobei
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-a-aminobuttersäure, anderen unnatürlichen Aminosäuren wie Phenylglycin, 2-Pyridylalanin, 3-Pyridylalanin, 4-Pyridylalanin, 1-Naphthyalanin oder 2-Naphthylalanin, gegebenenfalls mit Schutzgruppen, in ihrer D- oder L-Form oder gegebenenenfalls
- E und G: über eine Alkylkette -(CH₂)_{q} miteinander verknüpft sind,
- K: -CO-, -SO₂- oder -CH₂- sein kann,
- L: ein Carrier-System, Reporter-Ligand, eine löslichkeitsvermittelnde Gruppe oder Wasserstoff sein kann,
- M: unabhängig von L ein Carrier-System, Reporter-Ligand, eine löslichkeitsvermittelnde Gruppe oder Wasserstoff sein kann,
- m: 0, 1, 2 oder 3 sein kann,
- n: 0, 1, 2 oder 3 sein kann,
- p: 0 oder 1 sein kann,
- q: 0, 1 oder 2 sein kann, und
- s: Werte zwischen 3 und 20 annehmen kann.

Die Erfindung betrifft weiterhin Verbindungen der allgemeinen Formel (II) 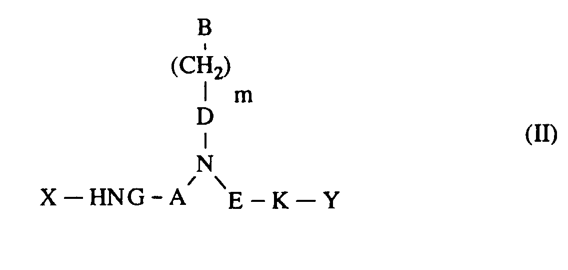 in der
- A: für -(CH₂)ₙ- oder -CO- steht,
- B: für alle natürlichen oder unnatürlichen Nukleobasen, wie z.B. Thymin, Uracil, Cytosin, Adenin, Guanin, Hypoxanthin, oder durch chemische Modifikation von diesen abgeleitete Derivate oder halogenierte Vorstufen von diesen, gegebenenfalls an den Aminogruppen mit Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert oder mit freien Aminogruppen steht,
- D: -(CO)ₚ- steht,
- E und G: unabhängig voneinander für -CHR- stehen,
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-α-aminobuttersäure, anderen unnatürlichen Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Iodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, in ihrer D- oder L-Form oder gegebenenenfalls
- E und G: über eine Alkylkette -(CH₂)_{q}- miteinander verknüpft sind,
- K: für -CO-, -SO₂- oder -CH₂- steht,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht und
- n: 0, 1, 2, 3 oder 4 sein kann und
- q: 0, 1 oder 2 sein kann.

Bevorzugt sind Verbindungen der allgemeinen Formel (II),
in der
- A: für -(CH₂)ₙ- oder -CO- steht,
- B: für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
- D: für -(CO)ₚ- steht,
- E und G: unabhängig voneinander für -CHR- stehen,
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-α-aminobuttersäure, anderen unnatürlichen Aminosäuren wie Phenylglycin, 2-Pyridylalanin, 3-Pyridylalanin, 4-Pyridylalanin, 1-Naphthylalanin oder 2-Naphthylalanin, gegebenenfalls mit Schutzgruppen, in ihrer D- oder L-Form oder gegebenenenfalls
- E und G: über eine Alkylkette -(CH₂)_{q} miteinander verknüpft sind,
- K: -CO-, -SO₂- oder -CH₂- sein kann,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht und
- n: 0, 1, 2 oder 3 sein kann und
- q: 0, 1 oder 2 sein kann.

Besonders bevorzugt sind Verbindungen der allgemeinen Formel (II),
in der
- A: für -(CH₂)ₙ- oder -CO- steht,
- B: für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
- D: für -(CO)ₚ- steht,
- E und G: unabhängig voneinander für -CHR- stehen,
- R: Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin oder Dehydroaminosäuren, wie z.B. Dehydroalanin, Dehydro-α-aminobuttersäure, gegebenenfalls mit Schutzgruppen, in ihrer D- oder L-Form oder gegebenenfalls
- E und G: über eine Alkylkette -(CH₂)_{q} miteinander verknüpft sind,
- K: -CO- ist,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht und
- n: 0, 1, oder 3 sein kann und
- q: 0 oder 1 sein kann.

Mit Carrier-System oder Reporter-Ligand ist gemeint ein zellspezifisches Bindungs- und Erkennungs-Agens, das spezifisch an die Zelloberfläche bindet und das die Internalisierung der der Erfindung zugrunde liegenden Nukleinsäuren-bindenden Oliogomeren bewirkt. Die Internalisierung kann auf verschiedenem Wege, z.B. durch Endocytose oder aktive Transportmechanismen erfolgen.

Die Struktur der Zelloberfläche kann ein Protein, Polypeptid, Kohlenhydrat, Lipid oder eine Kombiantion daraus sein. Typischerweise wird die Zellaufnahme durch Oberflächenrezeptoren bewirkt. Deshalb kann das Bindungs- und Erkennungs-Agens ein natürlicher oder synthetischer Ligand eines Rezeptors sein.

Der Ligand kann ein Protein, Polypeptid, Kohlenhydrat, Lipid oder eine Kombination von diesen sein, ausgestattet mit funktionellen Gruppen, die so angeordnet sind, daß sie durch die Zelloberflächen-Struktur erkannt werden können. Es kann sich auch um eine Komponente oder die Gesamtheit eines biologischen Organismus handeln, z.B. eines Virus, einer Zelle oder um artifizielle Transportsysteme, wie z.B. Liposomen. Es kann sich weiterhin um einen Antikörper oder ein Analogon eines Antikörpers handeln.

Für die Adressierung an unterschiedlichen Zellen müssen verschiedene Liganden eingesetzt werden.

Als Liganden für die Adressierung an Makrophagen kommen bevorzugt Kohlenhydrate, wie z.B. Mannose, Polykationen, wie z.B. Polylysine, Polyarginine, Polyornithine, basische Proteine, wie z.B. Avidin, sowie Glycopeptide, Peptide oder Lipopeptide in Frage (G.Y. Chu et al., WO 9304701).

Mit löslichkeitsvermittelnden Gruppen sind funktionelle Gruppen gemeint, die die Löslichkeit in Wasser vermitteln. Dabei kann es sich z.B. um Ester oder Amide von Aminosäuren, Hydroxycarbonsäuren, Aminosulfonsäuren, Hydroxysulfonsäuren oder Diaminen handeln. Bevorzugt sind Amide von Diamincarbonsäuren wie Ornithin, Lysin oder 2,4-Diaminobuttersäure.

### Peptid-Nukleinsäuren mit Glycylglycin-Backbone

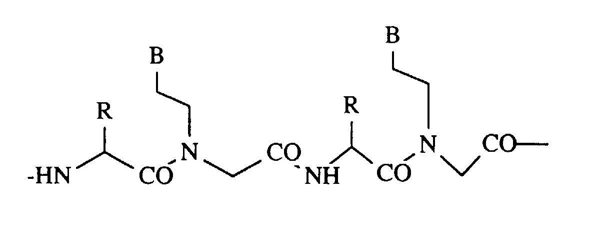

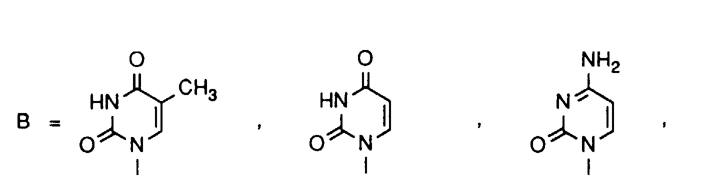

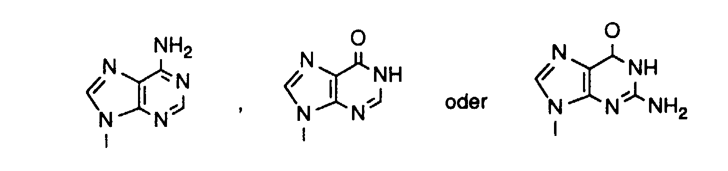

Bei den Verbindungen dieses Typs ist das Ribosephosphat- bzw. Desoxyribosephosphat-Backbone von RNA bzw. DNA durch ein Polyamid-Backbone aus Glycylglycin-Dipeptiden ersetzt (R = H). Das resultierende Oligomere zeichnet sich durch hohe Flexibilität aus. Außerdem ist durch den Einbau anderer Aminosäuren anstelle von Glycin in jeder zweiten Position (R ≠ H) eine Vielzahl weiterer Verbindungen mit unterschiedlichen Eigenschaften (z.B. Polarität und Ladungsverteilung) zugänglich.

### Peptid-Nukleinsäuren mit 4-Aminoprolin-Backbone

Bei diesem Strukturtyp ist das Ribose- bzw. Desoxyribosephosphat-Backbone der natürlichen Nukleinsäuren durch ein Polymeres aus 4-Aminoprolin-Basis ersetzt. Es resultieren Verbindungen geringer Flexibilität. Aufgrund der jeweils vorliegenden Konfiguration werden unterschiedliche Vororientierungen der Oligomeren erreicht.

(B ist definiert wie oben)

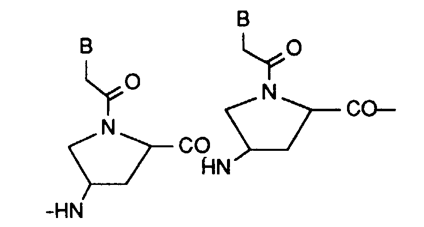

### Synthesen der monomeren Bausteine

### Monomere für Peptid-Nukleinsäuren mit Glycylglycin-Backbone

Die Synthese von Monomeren für Glycylglycin-Verbindungen wird am Beispiel des Thymin-Bausteines erläutert:

N-(Benzyl)ethanolamin 1 wird mit Bromessigsäure-tert.-butylester 2 in Gegenwart einer Hilfsbase alkyliert zu 3. Die Hydroxylgruppe wird in eine Abgangsgruppe überführt (z.B. zu 4) und gegen eine heterocyclische Nukleobase (z.B. zu 5) substituiert.

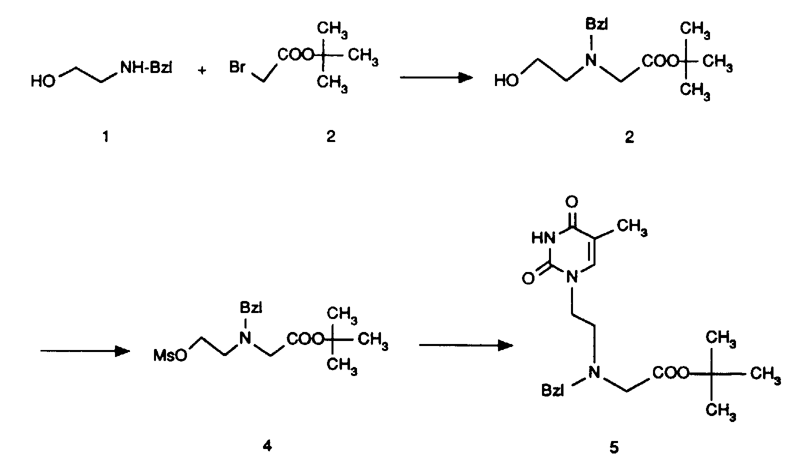

Die N-Benzylgruppe wird hydrogenolytisch entfernt und das resultierende Amin, z.B. 6, anschließend mit N-Fmoc-glycin in Gegenwart eines Kondensationsmittels wie N,N'-Dicyclohexylcarbodiimid zur Reaktion gebracht. Der resultierende Dipeptidester 7 wird mit Trifluoressigsäure gespalten. Das Produkt 8 ist für den Einsatz in der Peptid-Festphasensynthese unter "Fmoc-Bedingungen" geeignet.

Die Synthese der Derivate anderer Nukleobasen ist analog möglich.

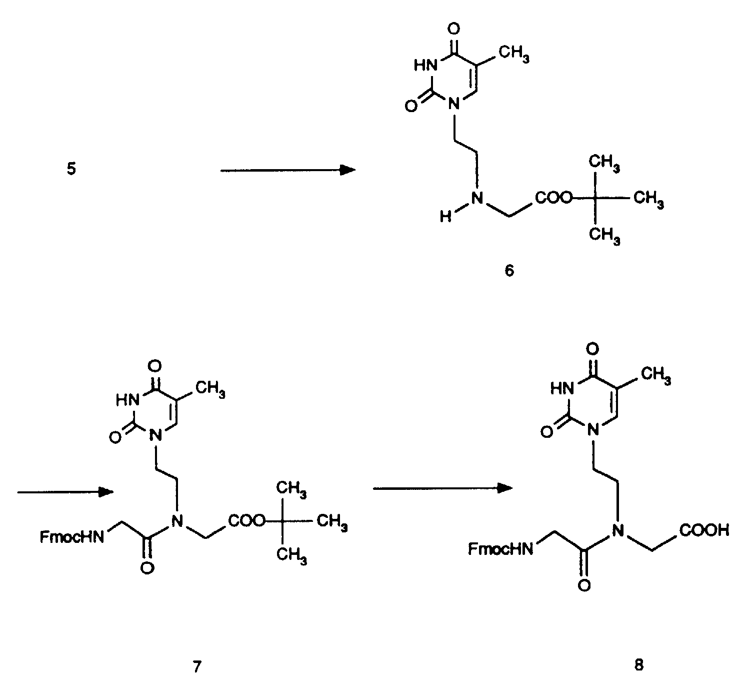

### Monomere für Peptid-Nukleinsäuren mit 4-Aminoprolin-Backbone

Die Synthese der Monomeren für diesen Strukturtyp sei am Beispiel des Thymin-Derivates der L-cis-Reihe erläutert:

trans-N-(Benzyloxycarbonyl)4-hydroxy-L-prolin 9 wird mit Methyliodid und Cäsiumcarbonat in den Methylester 10 überführt. Die 4-Hydroxygruppe in 10 wird in eine Abgangsgruppe, z.B. ein Methylsulfonat, umgewandelt. Dabei entsteht z.B. 11. Die Substitution mit Natrium- oder Lithiumazid führt unter Inversion an C-4 zum cis-4-Azido-derivat 12. Die Reduktion der Azidfunktion zur Aminogruppe in Gegenwart der N-(Benzyloxycarbonyl)-Schutzgruppe gelingt z.B. mit Schwefelwasserstoff in Pyridin/Wasser. Anschließende Einführung einer tert.-Butoxycarbonyl-Schutzgruppe ergibt das selektiv entblockierbare Derivat 13.

Die α-N-(Benzyloxycarbonyl)-Schutzgruppe der Verbindung 13 kann hydrogenolytisch entfernt werden. Dabei entsteht 14, das für die Verknüpfung mit Nukleobasen geeignet ist. So ergibt z.B. die Umsetzung von 14 mit 1-Carboxymethyl-thymin 15 in Gegenwart von Kondensationsmitteln, wie z.B. N,N'-Dicyclohexylcarbodiimid, die Thymin-Verbindung 16. Die basische Verseifung des Methylester führt zur Peptid-Nukleinsäure 17, die für den Einsatz in der Peptid-Festphasensynthese geeignet ist.

Führt man an 10 eine doppelte Inversion durch, so erhält man auf analogem Weg Derivate des L-trans-4-Aminoprolins. Ausgehend von D-Hydroxyprolin sind Verbindungen der D-Reihe zugänglich.

Die Derivate anderer Nukleobasen sind auf analogen Wegen herstellbar.

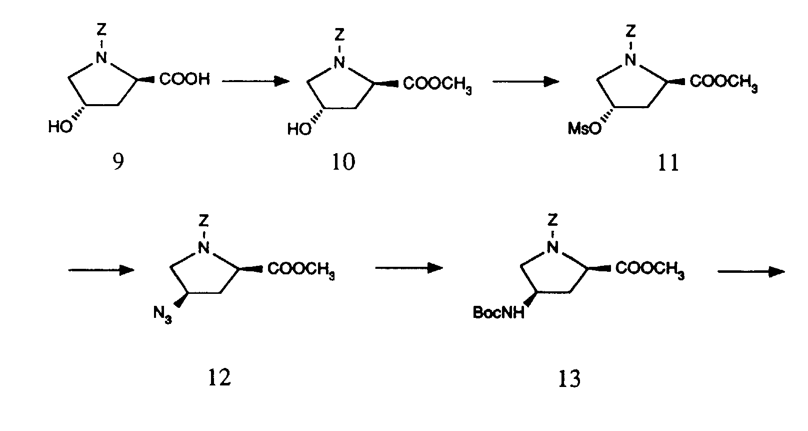

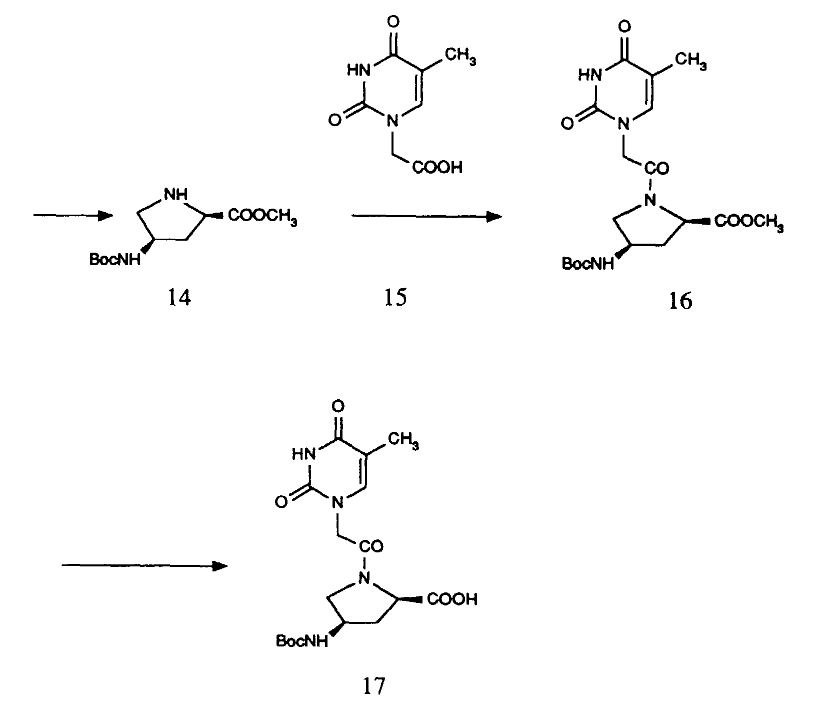

### Oligomerisierung

Die Verknüpfung der Bausteine zu Oligomeren erfolgt durch Festphasensynthese, vorzugsweise an einem Applied Biosystem 431-A Peptidsynthesizer. Als polymere Träger wurden PAM-, MBHA- oder HMP-Resins der Firma Applied Biosystems eingesetzt. Die Bausteine werden in Analogie zur konventionellen Peptidsynthese entweder nach dem Fmoc- oder Boc-Verfahren verknüpft. Die Aktivierung der Bausteine erfolgt in n-Methyl-2-pyrrolidon durch Umsetzung mit Hydroxybenzotriazol/Dicyclohexylcarbodiimid oder Pentafluorphenol/Dicyclohexylcarbodiimid. Die Sequenzen werden im Anschluß durch Behandlung mit HF oder Trifluormethansulfonsäuren (Boc-Methode, PAM- oder MBHA-Resin) oder durch Trifluoressigsäure (Fmoc-Methode, HMP-Resin) abgespalten. Die Reaktionsprodukte werden durch präparative HPLC an RP 8 mit einem ansteigenden Gradienten von Trifluoressigsäure in Acetonitril/Wasser isoliert. Nach dieser Methode wurden Sequenzen mit Kettenlängen bis 15 synthetisiert.

Zur Oligomerisierung wurden vorzugsweise nachfolgend aufgeführte Glycylglycin-Bausteine eingesetzt:

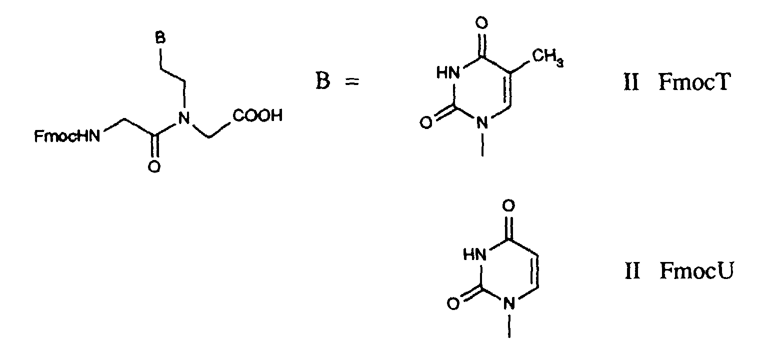

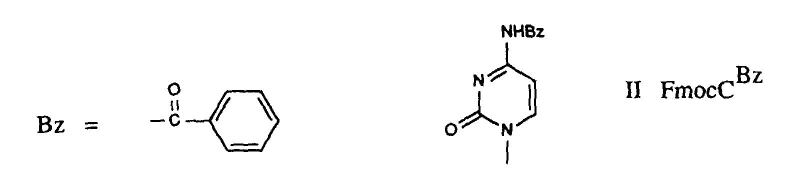

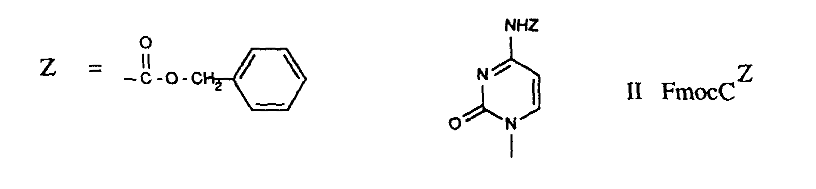

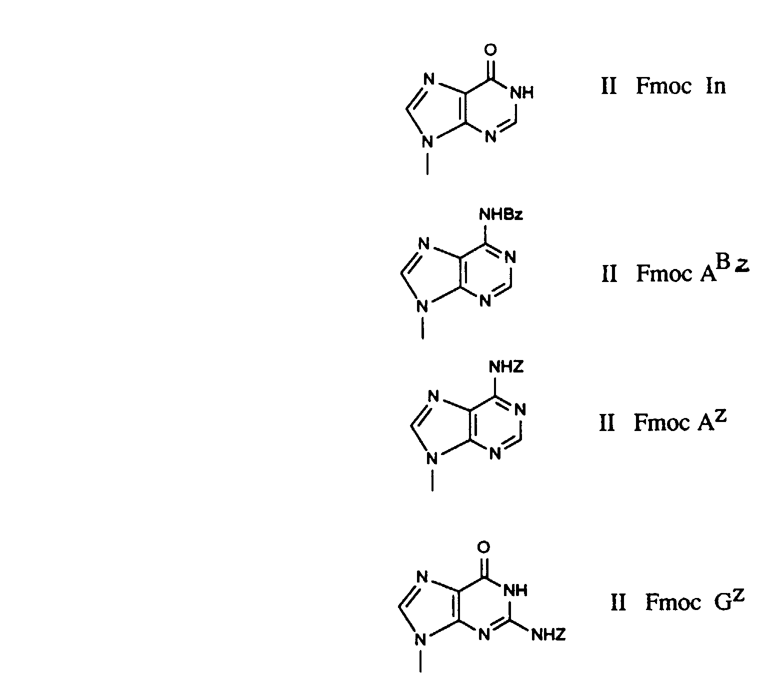

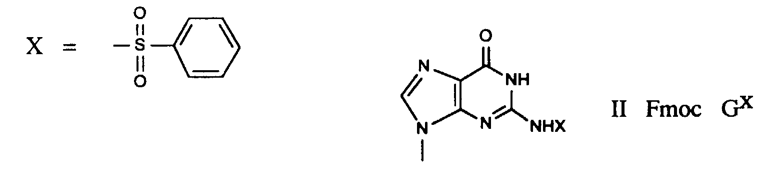

Dies ergibt folgende Glycylglycin-Syntheseäquivalente:

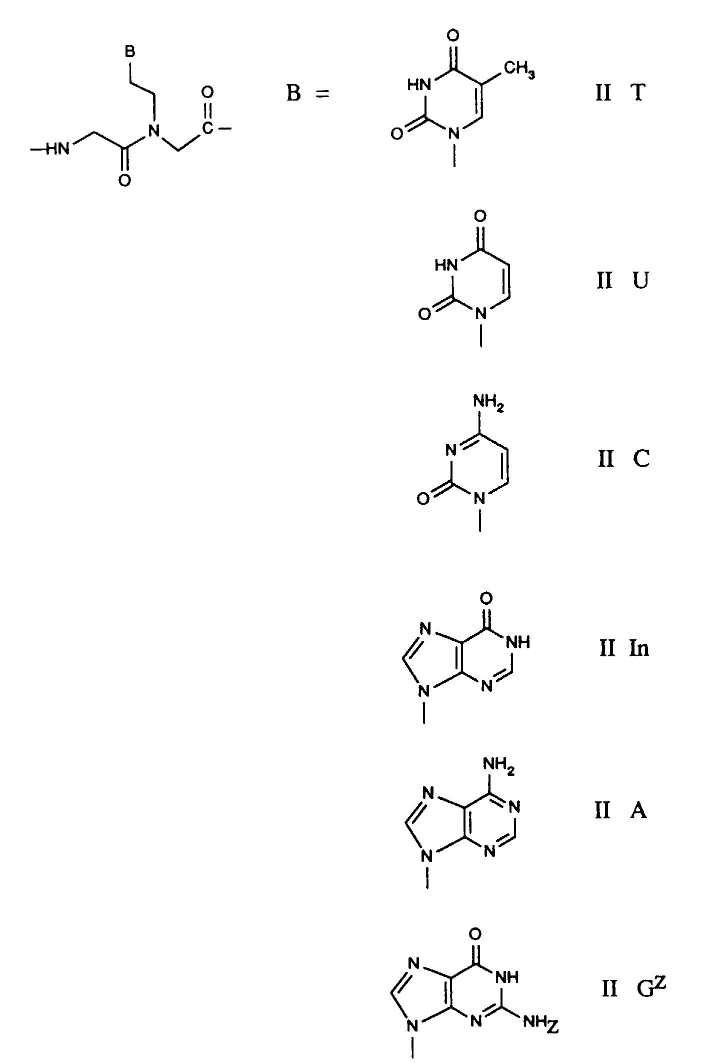

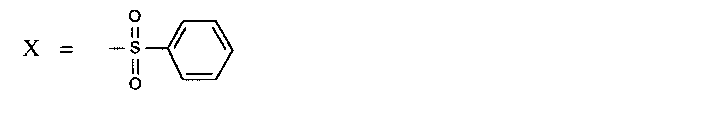

Zur Oligomerisierung wurden vorzugsweise nachfolgend aufgeführte 4-Aminoprolin-Bausteine eingesetzt:

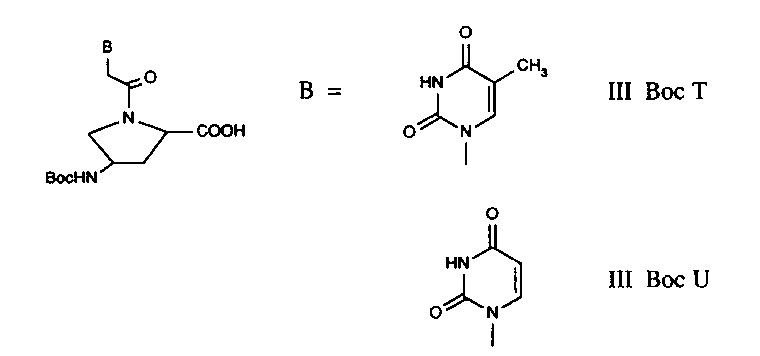

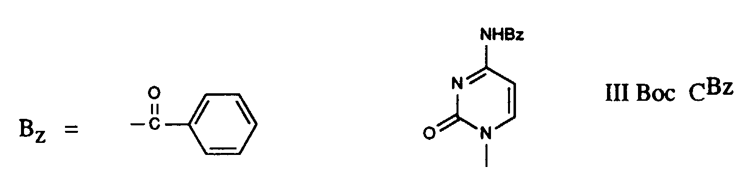

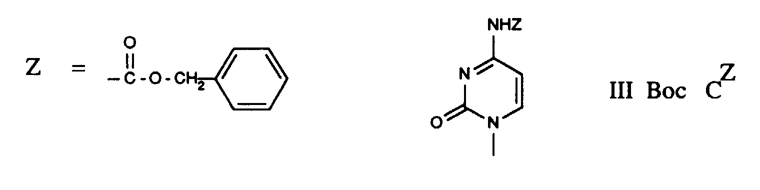

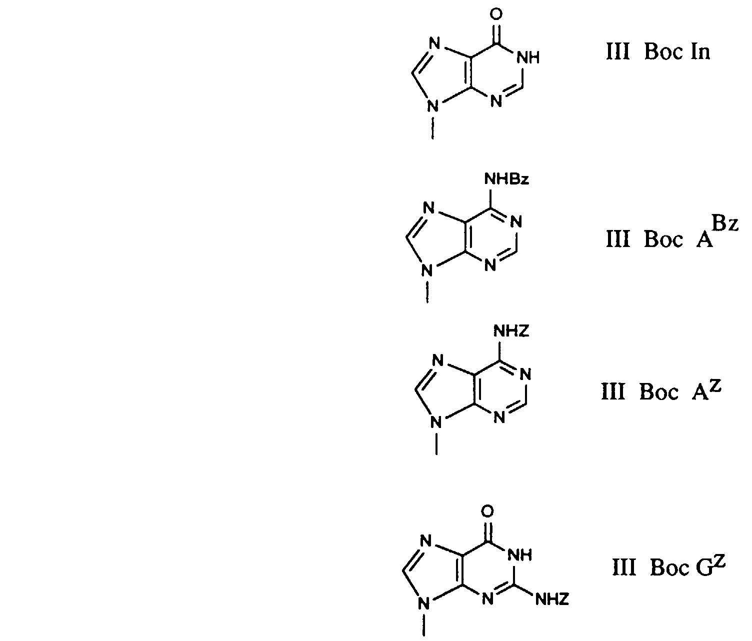

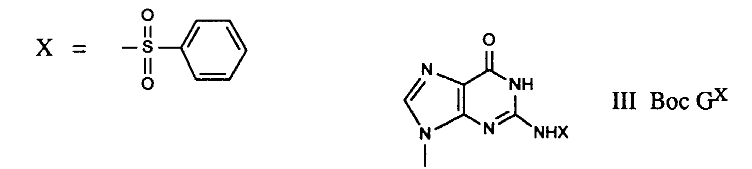

Dies ergibt folgende 4-Aminoprolin-Syntheseäquivalente:

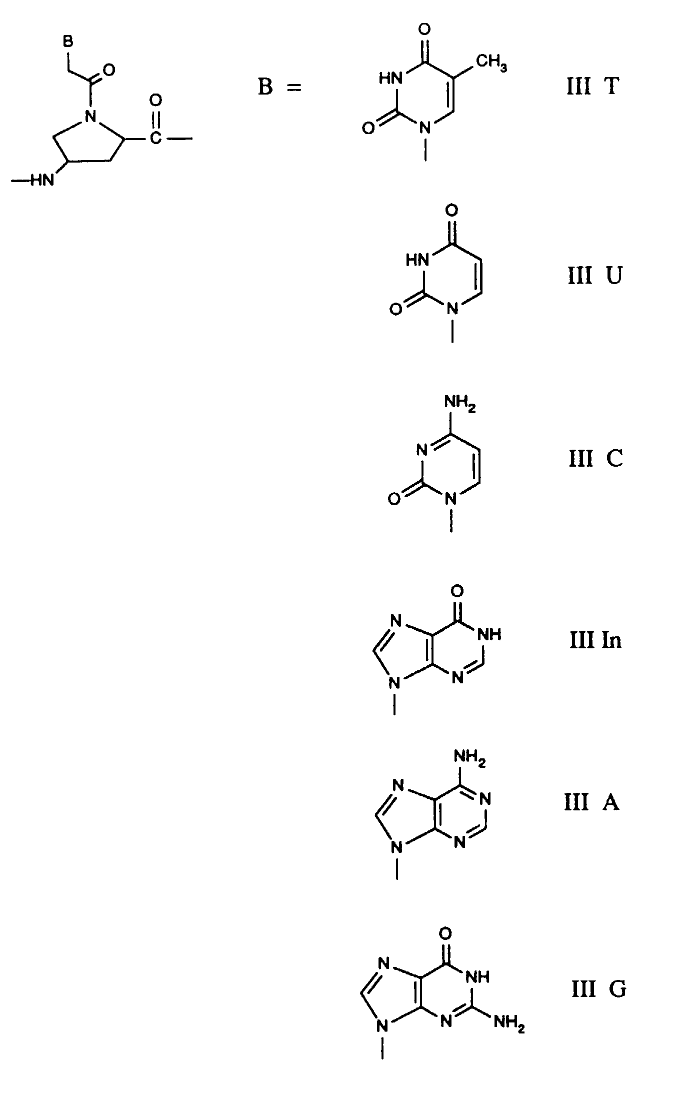

### Protease- und Nukleasestabilität von Nukleinsäure bindenden Polymeren

Neben der Kettenlänge, der Sequenz und der Zellpermeabilität spielt die Protease- und Nukleaseresistenz, eine wichtige Rolle für die biologische Wirkung von Nukleinsäuren-bindenden Oligomeren.

Die synthetisierten Oligomere vom Aminoprolin-Typ wurden deshalb in bezug auf ihre Protease- und Nukleasestabilität mit natürlichen Oligonukleotiddiestern verglichen.

Die Nukleinsäuren-bindenden Oligomere wurden hierzu mit unspezifischen und spezifischen Proteasen wie z.B. Pronase E, Proteinase K, Trypsin, Endoprotease Lys.C, V8 Protease, Protease IX, Protease XXI, Nukleasen wie z.B. S1 Nuklease, Ba131 Nuklease, Phosphodiesterase sowie Zellextrakten, Organextrakten und Blutserum und Blutextrakten, die verschiedene Nukleasen und Proteasen enthalten, behandelt. Durch Polyacrylamidgelelektrophorese und UV-Shadowing auf DC-Platten mit UV-Indikator sowie durch Silberanfärbung der Polyacrylamidgele wurden die Oligomere auf Degradation überprüft.

Natürliche Oligonukleotid-Diester weisen eine nur geringe Nukleasestabilität auf. Sie werden innerhalb von 30 Minuten bis zu 1 Stunde vollständig degradiert.

Nukleinsäuren-bindende Oligomere des Aminoproplin-Typs sind dagegen vollständig resistent gegenüber Nukleasen und Proteasen und eignen sich deshalb besonders gut für den Einsatz als Antisense-Inhibitoren.

### Untersuchungen zur Bindung an Nukleinsäuren

### DNA-Einzelstrangbindung durch Gel-shift-Analysen

Die hier beschriebenen Nukleinsäure-bindenden Oligomere wurden in Gel-shift-Analysen untersucht. Bei diesen Band-shift-Experimenten wurde das geänderte Laufverhalten von radioaktiv-markierten DNA-Diesteroligonukleotiden nach Hybridisierung mit den hier beschriebenen Oligomeren in einer Polyacrylamidgelelektrophorese gemessen. Die hybridisierten DNA-Diesteroligonukleotide wandern in der Elektrophorese langsamer, weil sich durch die Hybridbildung erstens das Molekulargewicht vergrößert und zweitens sich die relative Ladung pro Masseeinheit verringert. Im Vergleich zu einem nicht hybridisierten DNA-Oligonukleotid kommt es im Gel zu einem verzögerten Laufverhalten (Gel-retardation).

### Strangverdrängung in doppelsträngiger Plasmid-DNA

Nukleinsäure-bindende Oligomere wie z.B. solche mit einem 4-Aminoprolin- oder Glycylglycin-Backbone sind biologisch aktiv, indem sie sequenzselektiv die Bindung durch Strangverdrängung an doppelsträngige DNA (ds DNA) zeigen. Diese Wirkung von nukleinsäurebindenden Oligomeren läßt sich sequenzabhängig und konzentrationsabhängig in in-vitro Tests nachweisen.

### Inhibition der Genexpression (in-vitro Translationstest)

Nukleinsäure-bindende Oligomere, die in Gel-shift- und Strangverdrängungs-Experimenten aufgefallen sind, wurden auf ihre Fähigkeit getestet, die Proteinsynthese spezifischer Gene zu inhibieren. Voraussetzung dafür ist, daß in dem betreffenden Gen die entsprechende Sequenz des Nukleinsäure-bindenden Oligomeren in paralleler oder antiparalleler Basensequenz enthalten ist und daß eine geeignete Zielsequenz in dem zu hemmenden Gen durch Vorversuche z.B. durch Diesteroligonukleotide ausgewählt wird. Es hatte sich bei den in-vitro Translationen gezeigt, daß die hier beschriebenen Nukleinsäure-bindenden Oligomeren sehr potente, sequenzspezifische Inhibitoren für Genexpressionen sind. Dabei waren kürzere Sequenzen und geringere Konzentrationen im Vergleich zu Diesteroligonukleotiden ausreichend.

Therapeutisch wirksame Nukleinsäuren-bindende Oligomere, wie sie hier beschrieben sind, können nicht nur wie oben erwähnt die Genexpression durch die sequenz-selektive Bindung in RNA hemmen, sondern natürlich auch aufgrund ihrer Eigenschaft doppelsträngige DNA verdrängen, die Promotor- und Enhancersequenzen von zu hemmenden Genen sequenz-selektiv inaktivieren.

Nukleinsäuren-bindende Oligomere enthalten für diese Anwendung der Gen-Inaktivierung nicht nur Nukleobasensequenzen von (-)-Strang DNA sondern durchaus auch die (+)-Strang DNA Sequenz oder zu hemmenden Ziel-DNA.

Die Zielsequenz kann aus dem Promotor eines krankheitsauslösenden Gens stammen. Insbesondere Enhancer- oder Transkriptionsfaktoren und DNA- oder RNA-Polymerase bindende Zielsequenzen aus Genen von Viren, Bakterien, Pilzen, Endoparasiten, Oncogenen, oder Genen die an der Ausprägung von Entzündungserkrankungen, Autoimmunerkrankungen oder Herzkreislauferkrankungen wie Bluthochdruck oder Arteriosklerose beteiligt sind, sind hier als mögliche Zielsequenzen für die therapeutische Anwendung von Nukleinsäuren-bindenden Oligomeren zu nennen.

Die entsprechenden pharmazeutischen Zubereitungen enthalten neben den Nukleinsäuren-bindenden Oligomeren die für parenterale Zubereitungen üblichen Hilfsstoffe, wie z.B. Puffer und/oder Stabilisatoren oder Liposomenformulierungen. Ferner ist eine topische Anwendung denkbar. Dier hierfür einsetzbaren Zubereitungen sind z.B. Salben, Cremes, Lösungen oder Pflaster, die neben dem Wirkstoff die für diese Anwendung geeigneten pharmazeutischen Hilfsstoffe enthalten.

### Beispiele

### Beispiel 1

### N-Benzyl-N-(2-hydroxyethyl)-glycin-tert.-butylester (3)

Zu einer Lösung von N-Benzylethanolamin (30,2 g; 0,2 mol) und Triethylamin (27,9 ml; 0,2 mol) in wasserfreiem N,N-Dimethylformamid (200 ml) wird unter Eiskühlung Bromessigsäure-tert.-butylester (32,3 ml; 0,2 mol) langsam hinzugetropft. Das Gemisch wird 22 h bei Raumtemperatur gerührt und danach in vacuo eingeengt. Der Rückstand wird mehrfach mit Toluol nachdestilliert. Das resultierende Öl wird in Dichlormethan (400 ml) aufgenommen und zweimal mit Wasser (je 160 ml) ausgeschüttelt. Die organische Phase wird getrocknet (Magnesiumsulfat) und eingeengt.
Ausbeute: 47,8 g (90 %), farbloses Öl.

### Beispiel 2

### N-Benzyl-N-[2-(methansulfonyloxy)ethyl]-glycin-tert.-butylester (4)

Das Produkt aus Beispiel 1 (10,0 g; 38 mmol) wird in wasserfreiem Pyridin (185 ml) gelöst und bei 0°C Methansulfonsäurechlorid (3,7 ml; 46 mmol) langsam zugetropft. Die Lösung wird 6,5 h bei Raumtemperatur gerührt. Anschließend wird mit Dichlormethan (740 ml) verdünnt und zweimal mit 10 %iger Natriumhydrogencarbonat-Lösung (je 250 ml) extrahiert. Die organische Phase wird getrocknet (Magnesiumsulfat), eingeengt und mehrfach mit Toluol nachdestilliert.
Ausbeute: 10,79 g (85 %), braunes Öl.

### Beispiel 3

### N-Benzyl-N-[2-(thymin-1-yl)ethyl]-glycin-tert.-butylester (5)

Das Produkt aus Beispiel 2 (10,73 g; 31 mmol), Thymin (7,88 g; 62 mmol) und Kaliumcarbonat (8,64g 62 mmol) werden in wasserfreiem N,N-Dimethylformamid (325 ml) suspendiert. Die Suspension wird 1 h bei Raumtemperatur und anschließend 6 h bei 80°C gerührt. Das abgekühlte Gemisch wird mehrfach mit Toluol co-destilliert, der Rückstand in Chloroform (500 ml) aufgenommen und zweimal mit Wasser (je 150 ml) extrahiert Das Rohprodukt wird durch Chromatographie an Kieselgel gereinigt (Laufmittel: Toluol/Ethanol 27:1).
Ausbeute: 5,58 g (48 %).

### Beispiel 4

### N-(2-Hydroxyethyl)-glycin-tert.-butylester

Die Darstellung erfolgt analog Beispiel 1 aus Ethanolamin (18,33 g; 0,3 mol) und Bromessigsäure-tert.-butylester (58,6 g; 0,3 mol) in Gegenwart von Triethylamin (30,7 g; 0,3 mol) in wasserfreiem N,N-Dimethylformamid (300 ml). Das Rohprodukt wird durch Chromatographie an Kieselgel gereinigt (Laufmittel: Chloroform/Methanol 16:1).
Ausbeute: 33,65 g (64%).

### Beispiel 5

### N-Benzyloxycarbonyl-N-(2-hydroxyethyl)-glycin-tert.-butylester

Eine Lösung des Produktes aus Beispiel 4 (32,15 g; 0,18 mol) in Dioxan (1,0 l) und Wasser (0,5 l) wird mit Kaliumcarbonat (33,0 g; 0,19 mol) versetzt. Bei Raumtemperatur wird eine Lösung von Chlorameisensäurebenzylester (27,3 ml; 0,19 mol) in Dioxan (0,2 l) langsam zugetropft. Die Lösung wird 3,5 h bei Raumtemperatur gerührt und anschließend eingeengt. Der Rückstand wird in Dichlormethan (3,0 l) aufgenommen und mit Wasser (1,0 l) ausgeschüttelt. Die wässrige Phase wird fünfmal mit Dichlormethan reextrahiert. Die organischen Phasen werden vereinigt, getrocknet (Magnesiumsulfat) und eingeengt.
Ausbeute: 24,47 g (43 %), hellgelbes Öl.

### Beispiel 6

### N-Benzyloxycarbonyl-N-[2-(methansulfonyloxy)ethyl]-glycin-tert.-butylester

Das Produkt aus Beispiel 5 (14,4 g; 47 mmol) wird in wasserfreiem Pyridin (270 ml) mit Methansulfonsäurechlorid (4,47 ml; 58 mmol) umgesetzt und aufgearbeitet, wie im Beispiel 2 beschrieben.
Ausbeute: 14,7 g (82 %), braunes Öl.

### Beispiel 7

### N-Benzyloxycarbonyl-N-(2-thymin-1-yl)-glycin-tert.-butylester

Das Produkt aus Beispiel 6 (8,47 g; 22 mmol) wird nach dem im Beispiel 3 angegebenen Verfahren mit Thymin (5,51 g; 44 mmol) in Gegenwart von Kaliumcarbonat (6,06 g; 44 mmol) in N,N-Dimethylformamid (220 ml) als Lösungsmittel umgesetzt. Die chromatographische Reinigung erfolgt mit Toluol/Ethanol (15:1) als Laufmittel.
Ausbeute: 3,19 g (35 %)

### Beispiel 8

### N-[2-(Thymin-1-yl)ethyl]-glycin-tert.-butylester (6)

a) Das Produkt aus Beispiel 3 (5,56 g; 15 mmol) wird in wasserfreiem Methanol (75 mol) bei Raumtemperatur und Normaldruck über Palladium/Aktivkohle (10 %; 2,76 g) 5 h hydriert. Die Lösung wird vom Katalysator abgesaugt und in vacuo eingeengt.
   Ausbeute: 3,82 g (91 %).
b) Das Produkt aus Beispiel 6 (2,76 g; 7 mmol) wird in Methanol (108 ml)/Dioxan (7 ml) bei Raumtemperatur und Normaldruck über Palladium auf Bariumsulfat (5 %; 1,38 g) 21 h hydriert. Nach dieser Zeit wird nochmals die gleiche Katalysatormenge zugesetzt und weitere 7,5 h hydriert, Anschließend wird wie unter a) aufgearbeitet.
   Ausbeute: 1,9 g (quantitativ)

### Beispiel 9

### N-[N'-(Fluorenylmethyloxycarbonyl)glycyl]-N-[2-(thymin-1-yl)ethyl]-glycin-tert.-butylester (7)

Das Produkt aus Beispiel 8 (3,78 g; 13 mmol) und N-(Fluorenylmethyloxycarbonyl)glycin (5,9 g; 20 mmol) werden unter Argon-Schutzgasatmosphäre in wasserfreiem N,N-Dimethylformamid (160 ml) gelöst. Bei 0°C wird N,N'-Dicyclohexylcarbodiimid (4,11 g; 20 mmol) portionsweise zugesetzt. Die Mischung wird 21 h bei Raumtemperatur gerührt und anschließend vom ausgefallenden Feststoff abgesaugt. Die Lösung wird in vacuo eingeengt, mehrfach mit Toluol nachdestilliert und das Rohprodukt an Kieselgel chromatopgrahiert.
(Laufmittel: Toluol/Ethanol 30:1 - 20:1). Ausbeute: 6,33 g (82 %).

### Beispiel 10

### N-[N'-(Fluorenylmethyloxycarbonyl)glycyl]-N-[2-(thymin-1-yl)ethyl]-glycin (8)

Der tert.-Butylester aus Beispiel 9 (6,31 g; 11 mmol) wird 7 h in 100 %iger Ameisensäure (105 ml) bei Raumtempatur stehengelassen. Die Lösung wird anschließend mit Toluol und zweimal mit Methanol (je 200 ml) co-destilliert. Das Produkt kristallisiert aus Methanol.
Ausbeute: 5,43 g (96 %).

### Beispiel 11

### N-Benzyloxycarbonyl-4-hydroxy-L-trans-prolinmethylester (10)

Eine Lösung von N-Benzyloxycarbonyl-4-hydroxy-L-trans-prolin **9** (47,5 g; 179 mmol) in wasserfreiem Methanol (900 ml) wird mit Cäsiumcarbonat auf pH = 9,0 - 9,5 eingestellt. Man rührt 30 min bei Raumtemperatur nach und engt ein. Nach 30minütiger Trocknung am Hochvakuum wird der Rückstand in wasserfreiem N,N-Dimethylformamid (900 ml) aufgenommen. Iodmethan (28,0 g; 197 mmmol) hinzugefügt und 21 h bei Raumtemperatur gerührt. Die Lösung wird eingeengt, mehrfach mit Toluol nachdestilliert, in Chloroform (1,8 l) aufgenommen, je einmal mit Wasser, 10 %iger Natriumhydrogencarbonat-Lösung und nochmals mit Wasser (je 600 ml) ausgeschüttelt. Die organische Phase wird getrocknet (Magnesiumsulfat) und eingeengt.
Ausbeute: 50,0 g (quantitativ).

### Beispiel 12

### N-Benzyloxycarbonyl-4-methansulfonyloxy-L-trans-prolinmethylester (11)

Das Produkt aus Beispiel 11 (50,0 g; 178 mmol) wird in wasserfreiem Pyridin (910 ml) gelöst. Unter Eiskühlung wird Methansulfonsäurechlorid (18,6 ml; 241 mmol) zugetropft. Anschließend wird 2,5 h nachgerührt, wobei die Lösung auf Raumtemperatur aufwärmen gelassen wird. Man verdünnt mit Dichlormethan (3,6 l) und schüttelt zweimal mit 10 %iger Natriumhydrogencarbonat-Lösung (je 1,1 l) aus. Die organische Phase wird getrocknet (Magnesiumsulfat), eingeengt und mehrfach mit Toluol nachdestilliert.
Ausbeute: 64,0 g (quantitativ).

### Beispiel 13

### 4-Azido-N-benzyloxycarbonyl-L-cis-prolinmethylester (12)

Das im Beispiel 12 erhaltene Methansulfonat (64,0 g; 178 mmol) wird in wasserfreiem N,N-Dimethylformamid (1,8 1) gelöst, Lithiumazid (43,8 g; 895 mmol) zugesetzt und 24 h bei 50°C gerührt. Die Reaktionslösung wird in vacuo eingeengt, mehrfach mit Toluol nachdestilliert, der Rückstand in Ethylacetat (1,8 1) aufgenommen und zweimal mit Wasser (je 600 ml) extrahiert. Die organische Phase wird getrocknet (Magnesiumsulfat) und eingeengt.
Ausbeute: 50,5 g (93 %).

### Beispiel 14

### N-Benzyloxycarbonyl-4-tert.-butyloxycarbonylamino-L-cis-prolinmethylester (13)

Eine Lösung der Azidoverbindung aus Beispiel 13 (52,0 g; 0,17 mol) in Pyridin/Wasser (5:1; 1,02 1) wird bei 0°C mit Schwefelwasserstoff gesättigt. Die Lösung wird 16 h bei Raumtemperatur belassen und anschließend in vacuo eingeengt. Der Rückstand wird in möglichst wenig Ethanol aufgenommen und mehrfach von ausfallenden Verunreinigungen abfiltriert. Die Ethanol-Lösung wird eingeengt und 16 h am Hochvakuum getrocknet. Das erhaltene Rohprodukt wird in Dioxan (850 ml) gelöst, mit Ethyldiisopropylamin (42,6 ml; 0,24 mol) und Di-tert.-butyldicarbonat (56,2 g; 0,26 mol) versetzt und 3 h bei Raumtemperatur gerührt. Das erhaltene Gemisch wird in vacuo eingeengt. Der Rückstand wird in Dichlormethan (1,7 1) aufgenommen und einmal mit 0,5 N Zitronensäurelösung (600 ml) ausgeschüttelt. Die wässrige Phase wird dreimal mit Dichlormethan reextrahiert. Die vereinigten organischen Phasen werden getrocknet (Magnesiumsulfat) und eingeengt. Das Rohprodukt wird durch Chromatographie an Kieselgel (Laufmittel: Toluol/Ethanol 22:1) gereinigt.
Ausbeute: 34,9 g (54 %).

### Beispiel 15

### 4-tert.-Butyloxycarbonylamin-L-cis-prolinmethylester (14)

Das Produkt aus Beispiel 14 (12,3 g; 32 mmol) wird in Methanol (320 ml) bei Raumtemperatur und Normaldruck 2,5 h über Palladium/Aktivkohle (10 %; 6,2 g) hydriert. Nach vollständiger Umsetzung wird vom Katalysator abgesaugt und eingeengt.
Ausbeute: 7,2 g (91 %).

### Beispiel 16

### 4-tert.-Butyloxycarbonylamino-N-[(thymin-1-yl-actyl]-L-cis-prolinmethylester (15)

Zu einer Lösung des Produktes aus Beispiel 15 (6,97 g; 29 mmol) in wasserfreiem N,N-Dimethylformamid (340 ml) wird 1-Carboxymethyl-thymin (7,82 g; 43 mmol) gegeben. Unter Eiskühlung wird sodann N,N'-Dicyclohexylcarbodiimid (8,82 g; 43 mmol) zugesetzt und 2,5 h nachgerührt, wobei sich die Lösung auf Raumtemperatur erwärmt. Vom ausgefallenen Feststoff wird abgesaugt, in vacuo eingeengt und mehrfach mit Toluol nachdestilliert. Das Rohprodukt wird chromatographisch gereinigt (Laumittel: Toluol/Ethanol 7:1).
Ausbeute: 10,44 g (89 %).

### Beispiel 17

### 4-tert.-Butyloxycarbonylamino-N-[(thymin-1-yl)acetyl]-L-cis-prolin (16)

Einer Lösung des Produktes aus Beispiel 16 (410 mg; 1,0 mmol) in Dioxan/Wasser (5:1; 7 ml) wird Lithiumhydroxid-Hydrat (50 mg; 1,2 mmmol) hinzugefügt und 5 h bei Raumtemperatur gerührt. Dann wird nochmals die gleiche Menge Lithiumhydroxid-Hydrat nachgesetzt und weiter 2 h bei Raumtemperatur belassen. Die Lösung wird mit 0,5 N Salzsäure neutralisiert und eingeengt. Das Produkt kristallisiert aus Methanol.
Ausbeute: 195 mg (49 %)

### Beispiel 18

### N⁴-Benzoyl-1-tert.-butyloxycarbonylmethylcyctosin

Zu einer Suspension von N⁴-Benzoylcytosin (21,5 g; 0,1 mol) und Kaliumcarbonat (13,8 g; 0,1 mol) in wasserfreiem N,N-Dimethylformamid (2,15 1) wird bei Raumtemperatur langsam Bromessigsäure-tert.-butylester (24 ml; 0,15 mol) zugetropft. Das heterogene Gemisch wird 20 h heftig bei Raumtemperatur verrührt, anschließend von unlöslichem Edukt abgesaugt, in vacuo eingeengt, mehrfach mit Toluol nachdestilliert, der Rückstand in Chloroform (1,0 1) aufgenommen, einmal mit Wasser (0,3 1) ausgeschüttelt und zügig die Phasen getrennt. Die organische Phase wird nochmals filtriert und eingeengt.
Ausbeute: 15,23 g (46 %).

### Beispiel 19

### N⁴-Benzoyl-1-carboxymethylcytosin

Das Produkt aus Beispiel 18 wird in Trifluoressigsäure (170 ml) gelöst und 1 h 45 min bei Raumtemperatur belassen. Anschließend wird fünfmal mit Toluol co-destilliert und das Produkt 24 h im Exsikkator über Phosphorpentoxid/Kaliumhydroxid getrocknet. Ausbeute: 11,8 g (93 %).

### Beispiel 20

### N-[(N⁴-Benzoylcyctosin-1-yl)acetyl]4-tert.-butyloxycarbonylamino-L-cis-prolinmethylester

Eine Lösung von N⁴-Benzoyl-1-carboxymethylcytosin (11,46 g; 42 mmol) und 4-tert.-butyloxycarbonylamino-L-cis-prolinmethylester(6,85 g; 28 mmol) in wasserfreiem N,N-Dimethylformamid (340 ml) wird unter Eiskühlung mit N,N'-Dicyclohexylcarbodiimid (8,67 g; 42 mmol) versetzt und 2 h gerührt, wobei sich die Lösung auf Raumtemperatur erwärmt. Sodann wird vom ausgefallenden Feststoff abgesaugt, eingeengt und mehrfach mit Toluol nachdestilliert. Das Rohprodukt wird chromatographisch gereinigt (Laufmittel: Toluol/Ethanol 8:1).
Ausbeute: 3,80 g (27 %).

### Beispiel 21

### N-[(N⁴-Benzoylcyctosin-1-yl)acetyl]4-tert.-butyloxycarbonylamino-L-cis-prolin

Das Produkt aus Beispiel 20 (2,68 g; 5,4 mmol) wird in Methanol (54 ml) gelöst, mit 1 N Natronlauge (6,3 ml) versetzt und 18 h bei Raumtemperatur gerührt. Anschließend wird mit 0,5 N Salzsäure neutralisiert und eingeengt. Das Produkt kristallisiert aus Methanol.
Ausbeute: 2,44 g (94 %).

### Beispiel 22

### N-Benzyloxycarbonyl-4-nitrobenzoyloxy-L-cis-prolinmethylester

Es werden 3,29 g (11,8 mmol) Z-L-trans-Hydroxyprolinmethylester in 50 ml abs. THF gelöst. Dann gibt man nacheinander bei Raumtemperatur 3,76 g (14,3 mmol) Triphenylphosphin und 2,19 g (13,1 mmol) p-Nitrobenzoesäure zu. Man kühlt ab auf 0°C und tropft bei dieser Temperatur 2,5 g (14,3 mmol) DEAD in abs./THF zu. Anschließend rührt man über Nacht bei Raumtemperatur. Nun wird am Hochvakuum eingeengt und der Rückstand an Kieselgel (Laufmittel EE/Hexan 2:1) chromatographiert.
Ausbeute: 3,34 g (66,2 % d.Th.)
R_{f}: 0,68 Laufmittel EE/Hexan (2:1)

### Beispiel 23

### N-Benzyloxycarbonyl-4-hydroxy-L-cis-prolinmethylester

2,71 g (6,2 mmol) N-Benzyloxycarbonyl-4-nitrobenzoyloxy-L-cis-prolinmethylester werden in 600 ml abs. Methanol gelöst. Anschließend tropft man eine Lösung von 0,34 g (6,3 mmol) Natriummethylat in CH₃OH innerhalb von 5 min. zu. Es wird 30 min. bei Raumtemperatur verrührt und danach verdünnte Salzsäure zugesetzt bis der pH-Wert 5-6 beträgt. Man engt ein, versetzt den Rückstand mit gesättigter Natriumchlorid-Lösung und schüttelt 2x mit Essigsäureethylester aus. Die organische Phase wird über Na₂SO₄ getrocknet, abfiltriert, eingeengt und über Kieselgel (Laufmittel EE/Hexan 2:1) chromatographiert.
Ausbeute: 1,52 g (86,0 % d.Th.)
R_{f}: 0,28 Laufmittel EE/Hexan (2:1)

### Beispiel 24

### N-Benzyloxycarbonyl-4-methansulfonyloxy-L-cis-prolinmethylester

1,59 g (5,7 mmol) N-Benzyloxycarbonyl-4-hydroxy-L-cis-prolinmethylester werden in 50 ml abs. Pyridin gelöst und auf 0°C abgekühlt. Bei 0°C tropft man 0,6 ml (7,7 mmol) Methansulfonsäurechlorid zu und rührt anschließend 3 Stunden bei Raumtemperatur. Nun engt man ein und versetzt danach den Rückstand mit 100 ml Methylenchlorid, schüttelt 2x mit 10 %iger NaHCO₃-Lösung aus, trocknet über Natriumsulfat und engt wiederum ein, wobei man mehrfach mit Toluol nachdestilliert.
Ausbeute: quantitativ
R_{f}: 0,33 Laufmittel Toluol/EtOH (10:1)

### Beispiel 25

### 4-Azido-N-benzyloxycarbonyl-L-trans-prolinmethylester

2,01 g (5,6 mmol) N-Benzyloxycarbonyl-4-methan-sulfonyloxy-L-cis-prolinmethylester löst man in 50 ml abs. DMF und versetzt mit 1,37 g (28 mmol) Lithiumazid bei Raumtemperatur. Danach wird 24 h bei 50°C verrührt. Nun wird im Vakuum eingeengt und mehrfach mit Toluol nachdestilliert. Den Rückstand nimmt man in Essigsäureethylester auf und extrahiert 2 x mit Wasser, trocknet die organische Phase über Na₂SO₄ und engt wieder ein.
Ausbeute: 1,66 g (97 % d.Th.)
R_{f}: 0,58 Laufmittel Toluol/EtOH (10:1)

### Beispiel 26

### N-Benzyloxycarbonyl-4-tert.-butoxycarbonylamino-L-trans-prolinmethylester

Eine Lösung von 1,59 g (5,2 mmol) 4-Azido-N-benzyloxycarbonyl-L-trans-prolinmethylester in 27 ml Pyridin und 5,3 ml Wasser wird bei 0°C mit Schwefelwasserstoff gesättigt. Man läßt 45 min. bei Raumtemperatur verrühren, treibt überschüssiges H₂S mit Stickstoff aus und engt im Vakuum ein, wobei man mehrfach mit Toluol nachdestilliert. Den Rückstand nimmt man in 30 ml Ethanol auf und filtriert ausfallende Verunreinigungen ab. Die Mutterlage wird wieder eingeengt und über Nacht am Hochvakuum getrocknet. Das erhaltene Rohprodukt löst man in 30 ml Dioxan (abs.) versetzt mit 1,32 ml Ethyldiisopropylamin und 1,73 g BOC₂O. Dann wird 3 Std. bei Raumtemperatur verrührt und das erhaltene Gemisch im Vakuum eingeengt. Der Rückstand wird in 50 ml CH₂Cl₂ aufgenommen, einmal mit 0,5 N Zitronensäure ausgeschüttelt und die wässrige Phase wird noch 3 x mit CH₂Cl₂ nachextrahiert. Die vereinten organischen Phasen werden über Na₂SO₄ getrocknet, abfiltriert, eingeengt und das zurückbleibende Öl an Kieselgel (Laufmittel Toluol/EtOH 25:1) chromatographiert.
Ausbeute: 1,65 g (83,7 % d.Th.)
R_{f}: 0,44 Laufmittel Toluol/EtOH (10:1)

### Beispiel 27

### 4-tert.-Butyloxycarbonylamino-L-trans-prolinmethylester

2,95 g (7,7 mmol) N-Benzyloxycarbonyl4-tert.-butyloxycarbonylamino-L-transprolinmethylester werden in 80 ml abs. Methanol gelöst und bei Raumtemperatur und Normaldruck 2 1/2 Std. über Palladium/Aktivkohle (10 % 1,45 g) hydriert. Nach vollständiger Umsetzung (DC-Kontrolle Toluol/EtOH 8:1) wird vom Katalysator abgesaugt und eingeengt.
Ausbeute: 1,75 g (92,1 % d.Th.)
R_{f}: 0,23 Laufmittel Toluol/EtOH (8:1)

### Beispiel 28

### 4-tert.-Butyloxycarbonylamino-N-[(thymin-1-yl)-acetyl]-L-trans-prolinmethylester

275 mg (1,5 mmol) 1-Carboxy-methylthymin werden in 10 ml abs. DMF gelöst, auf -30°C gekühlt und bei dieser Temperatur mit 230 mg (1,7 mmol) HOBT x H₂O und 330 mg (1,7 mmol) EDCl x HCl versetzt. Dann rührt man 15 min bei -30°C nach und gibt eine Suspension von 245 mg (1 mmol) 4-tert.-Butyloxycarbonylamino-L-trans-prolinmethylester in 10 ml DMF und 0,41 ml (3 mmol) Triethylamin bei -30°C zu. Eine Stunde wird bei -30°C verrührt und anschließend 24 h bei Raumtemperatur versetzt. Dann wird eingeengt, der Rückstand in Essigsäureethylester aufgenommen und jeweils mit 1 N HCl, gesättigter NaHCO₃-Lösung und gesättigter NaCl-Lösung extrahiert. Die organische Phase wird über Na₂SO₄ getrocknet, abfiltriert und eingeengt.
Ausbeute: 300 mg (73,1 % d.Th.)
R_{f}: 0,16 Laufmittel Toluol/EtOH (8:1)

### Beispiel 29

### 4-tert.-Butyloxycarbonylamino-N-[(thymin-1-yl)-acetyl]-L-trans-prolin

Zu einer Lösung von 410 mg (1 mmol) 4-tert.-Butyloxycarbonylamino-N-[(thymin-1-yl)-acetyl]-L-trans-prolinmethylester in 6 ml Dioxan und 1,2 ml H₂O werden 50 mg (1,2 mmol) LiOH x H₂O hinzugefügt und 5 Stunden bei Raumtemperatur gerührt. Danach wird nochmals 50 mg LiOH x H₂O zugesetzt und für weitere 2 Stunden gerührt. Die Lösung wird mit 0,5 N HCl neutralisiert, eingeengt, wobei mehrmals mit Toluol nachdestilliert wird, und der Rückstand mit Isopropanol aufgekocht, heiß abgesaugt und mit Ether gewaschen.
Ausbeute: 375 mg (91,4 % d.Th.)
R_{f}: 0,24 Laufmittel CH₂Cl₂/CH₃OH (2:1)

### Beispiel 30

### 4-tert.-Butyloxycarbonylamino-N-[(N⁴-benzyloxycarbonylcytosin-1-yl)-acetyl]-L-cis-prolinmethylester

454 mg (1,5 mmol) 4-tert.-Butyloxycarbonylamino-L-cis-prolinmethylester werden in 10 ml abs. DMF gelöst und auf -30°C gekühlt. Bei dieser Temperatur gibt man 230 mg (1,7 mmol) HOBT und 330 mg (1,7 mmol) EDC zu und rührt 15 min. bei -30°C nach. Dann wird eine Lösung von 245 g N⁴-Benzyloxycarbonyl-1-carboxymethylcytosin in 10 ml abs. DMF und 0,41 ml (3 mmol) Triethylamin zugetropft (bei -30°C) und die Suspension 1 Stunde bei dieser Temperatur nachgerührt. Man läßt auf Raumtemperatur erwärmen und rührt über Nacht. Die Lösung wird nun am Hochvakuum eingeengt und mit Essigsäureethylester versetzt. Danach extrahiert man 1 x mit 1 N HCl, 1 x mit Na-Bicarbonatlösung und 1 x mit NaCl-Lösung. Der organische Teil wird über Na₂SO₄ getrocknet, abfiltriert und eingeengt.
Ausbeute: 460 mg (86,9 % d.Th.)
R_{f}: 0,57 Laufmittel CH₂Cl₂/CH₃OH (10:1)

### Beispiel 31

### 4-tert.-Butyloxycarbonylamino-N-[(N⁶-benzyloxycarbonylcytosin-9-yl)-acetyl]-L-cis-prolin

440 mg (0,83 mmol) 4-tert.-Butyloxycarbonylamino-N-[(N⁴-benzyloxycarbonylcytosin-1yl)-acetyl]-L-cis-prolinmethylester werden in 6 ml abs. Dioxan gelöst und 1,2 ml H₂O gelöst. Zu dieser Lösung gibt man 42 mg (1 mmol) LiOH x H₂O und verrührt 5 Stunden bei Raumtemperatur. Danach gibt man nochmals 42 mg LiOH x H₂O zu und verrührt für weitere 2 Stunden. Nun wird die Lösung mit 0,5 N HCl neutralisiert und anschließend eingeengt, wobei mehrmals mit Toluol redestilliert wird. Der kristalline Rückstand wird ca. 10 min. mit Isopropanol gekocht, heiß abgesaugt und mit Ether gewaschen.
Ausbeute: 250 mg (58,4 % d.Th.)
R_{f}: 0,57 Laufmittel CH₂Cl₂/CH₃OH (2:1)

### Beispiel 32

### 4-tert.-Butyloxycarbonylamino-N-[(N⁶-benzyloxycarbonyladenin-9-yl)-acetyl]-L-cis-prolinmethylester

327 mg (1,0 mmol) N⁶-Benzyloxycarbonyl-9-carboxymethyladenin werden in 610 ml abs. DMF gelöst und auf -30°C gekühlt. Bei dieser Temperatur gibt man 175 mg (1,3 mmol HOBT und 248 mg (1,3 mmol) EDCl zu und läßt weitere 15 min bei -30°C verrühren. Dann wird eine Lösung von 732 mg (3 mmol) 4-tert.-Butyloxycarbonylamino-L-cis-prolinmethylester in 10 ml abs. DMF und 0,41 ml (3 mmol) Triethylamin bei -30°C zugetropft. Es wird noch 1 Stunde bei -30°C nachgerührt und danach bei Raumtemperatur über Nacht gerührt. Nach dem Einengen am Hochvakuum versetzt man den Rückstand mit Essigsäureethylester und extrahiert 1 x mit 1 N HCl, 1 x mit Na-bicarbonatlösung und 1 x mit NaCl-Lösung. Die organische Phase wird getrocknet und über Na₂SO₄, abfiltriert und eingeengt.
Ausbeute: 360 mg (65 % d.Th.)
R_{f}: 0,86 Laufmittel CH₂Cl₂/CH₃OH (4:1)

### Beispiel 33

### 4-tert.-Butyloxycarbonylamino-N-[(N⁶-benzyloxycarbonyladenin-9-yl)-acetyl]-L-cis-prolin

4-tert.-Butyloxycarbonylamino-N-[(N⁶-benzyloxycarbonyladenin-9-yl)-acetyl]-L-cis-prolinmethylester 1,43 g (2,58 mmol) werden in 24 ml Dioxan und 4,8 ml H₂O gelöst. Zu dieser Lösung gibt man 126 mg (3,09 mmol) LiOH x H₂O und verrührt 5 Stunden bei Raumtemperatur. Danach gibt man nochmals 126 mg LiOH x H₂O zu und verrührt für weitere 2 Stunden. Nun wird die Lösung mit 0,5 N HCl neutralisiert und dann eingeengt; wobei mehrmals mit Toluol redestilliert wird. Der kristalline Rückstand wird ca. 10 min. mit Isopropanol gekocht, heiß abgesaugt und mit Ether gut gewaschen und getrocknet.
Ausbeute: 100 mg (43,1 % d.Th.)
R_{f}: 0,81 Laufmittel CH₂Cl₂/CH₃OH (8:1)

### Beispiel 34

### 4-tert.-Butyloxycarbonylamino-N-[(N⁶-benzyloxycarbonyladenin-9-yl)-acetyl]-L-trans-prolinmethylester

327 mg (1,0 mmol) N⁶-Benzyloxycarbonyl-9-carboxymethyladenin werden in 10 ml abs. DMF gelöst und auf -30°C gekühlt. Bei dieser Temperatur gibt man 175 mg (1,3 mmol) HOBT und 248 mg (1,3 mmol) EDCl zu und läßt weitere 15 min bei -30°C verrühren.
Dann wird eine Lösung von 732 mg (3 mmol) 4-tert.-Butyloxycarbonylamino-L-trans-prolinmethylester in 10 ml abs. DMF und 0,41 ml (3 mmol) Triethylamin bei -30°C eingetropft. Es wird noch 1 Stunde bei -30°C nachgerührt und danach bei Raumtemperatur über Nacht gerührt. Nach dem Einengen am Hochvakuum versetzt man den Rückstand mit Essigsäureethylester und extrahiert 1 x mit 1 N HCl, 1 x mit Na-bicarbonatlösung und 1 x mit NaCl-Lösung. Die organische Phase wird über Na₂SO₄ getrocknet, abfiltriert und eingeengt.
Ausbeute: 360 mg (65 % d.Th.)
R_{f}: 0,88 Laufmittel CH₂Cl₂/CH₃OH (4:1)

### Beispiel 35

### 4-tert.-Butyloxycarbonylamino-N[(N⁶-benzyloxycarbonyladenin-9-yl)acetyl]-L-trans-prolin

4-tert.-Butyloxycarbonylamino-N-[(N⁶-benzyloxycarbonyladenin-9-yl)-acetyl]-L-trans-prolinmethylester 1,22 g (2,20 mmol) werden in 20 ml Dioxan und 4 ml H₂O gelöst. Zu dieser Lösung gibt man 107 mg (2,64 mmol) LiOH x H₂O und verrührt 5 Stunden bei Raumtemperatur. Danach gibt man nochmals 107 mg LiOH x H₂O zu und verrührt für weitere 2 Stunden. Nun wird die Lösung mit 0,5 N HCl neutralisiert und dann eingeengt; wobei mehrmals mit Toluol redestilliert wird. Der kristalline Rückstand wird it Isopropanol gekocht, heiß abgesaugt und mit Ether gut gewaschen und getrocknet.
Ausbeute: 720 mg (61 % d.Th.)
R_{f}: 0,8 Laufmittel CH₂Cl₂/CH₃OH (4:1)

### Beispiel 36

### 4-tert.-Butyloxycarbonylamino-N-[(N4-benzyloxycarbonylcytosin-1-yl)-acetyl]-L-trans-prolinmethylester

454 mg (1,5 mmol) N4-Benzyloxycarbonyl- 1 -carboxymethylcytosin werden in 10 ml abs. DMF gelöst und auf -30°C gekühlt. Bei dieser Temperatur gibt man 230 mg (1,7 mmol HOBT und 330 mg (1,7 mmol) EDCl zu und rührt weitere 15 min bei -30°C nach. Dann wird eine Lösung von 245 mg (1 mmol) 4-tert.-Butyloxycarbonylamino-L-trans-prolinmethylester in 10 ml abs. DMF und 0,41 ml (3 mmol) Triethylamin bei -30°C zugetropft und die Suspension 1 Stunde bei dieser Temperatur nachgerührt. Nun läßt man auf Raumtemperatur erwärmen und rührt über Nacht. Die Lösung wird dann am Hochvakuum eingeengt und mit Essigsäureethylester versetzt. Danach extrahiert man 1 x mit 1 N HCl, 1 x mit Na-bicarbonatlösung und 1 x mit NaCl-Lösung. Der organische Teil wird über Na₂SO₄ getrocknet, abfiltriert und eingeengt. Der Rückstand wird durch Chromatographie über Kieselgel gereinigt. Laufmittel CH₂Cl₂/CH₃OH 10:1).
Ausbeute: 480 mg (90,7 % d.Th.)
R_{f}: 0,59 Laufmittel CH₂Cl₂/CH₃OH (10:1)

### Beispiel 37

### 4-tert.-Butyloxycarbonylamino-N-[(N⁴-benzyloxycarbonylcytosin-1-yl)-acetyl]-L-trans-prolin

450 mg (0,85 mmol) 4-tert.-Butyloxycarbonylamino-N-[(N4-benzyloxycarbonylcytosin-1-yl)-acetyl]-L-trans-prolinmethylester werden in 6 ml Dioxan und 1,2 ml H₂O gelöst. Zu dieser Lösung gibt man 42 mg (1 mmol) LiOH x H₂O und verrührt 5 Stunden bei Raumtemperatur. Danach gibt man nochmals 427 mg LiOH x H₂O zu und verrührt für weitere 2 Stunden. Nun wird die Lösung mit 0,5 N HCl neutralisiert und dann eingeengt; wobei mehrmals mit Toluol redestilliert wird. Der kristalline Rückstand wird mit Isopropanol gekocht, heiß abgesaugt und mit Ether gewaschen.
Ausbeute: 340 mg (77,6 % d.Th.)
R_{f}: 0,3 Laufmittel CH₂Cl₂/CH₃OH (2:1)

### Beispiel 38

### Festphasensynthese von H-(II T)₂-Ala-OH

192 mg (0,1 mmol) N-Fluorenylmethoxycarbonyl-alanin-HMP-Resin werden im Reaktionsgefäß vorgelegt. Die N-Fluorenylmethoxycarbonyl-Schutzgruppe wird vor jedem Kopplungsschritt durch Behandlung mit Piperidin abgespalten. Die Aktivierung von jeweils 253 mg (0,5 mmol) IIFmocT erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die N-Fluorenylmethoxycarbonyl-Schutzgruppe durch Behandlung mit Piperidin entfernt. Die Abspaltung vom Träger erfolgt durch 60 minütige Behandlung mit Trifluoressigsäure. Die Reindarstellung erfolgt durch RP-HPLC an C8 mit einem ansteigenden Gradienten von TFA in Acetonitril.
Ausbeute: 12 mg (19 %).

### Beispiel 39

### Festphasensynthese von H-(II T)₈-Ala-OH

192 mg (0,1 mmol) N-Fluorenylmethoxycarbonyl-alanin-HMP-Resin werden im Reaktionsgefäß vorgelegt. Die N-Fluorenylmethoxycarbonyl-Schutzgruppe wird vor jedem Kopplungsschritt durch Behandlung mit Piperidin abgespalten. Die Aktivierung von jeweils 253 mg (0,5 mmol) IIFmocT erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die N-Fluorenylmethoxycarbonyl-Schutzgruppe durch Behandlung mit Piperidin entfernt Die Abspaltung vom Träger erfolgt durch 60 minütige Behandlung mit Trifluoressigsäure. Die Reindarstellung erfolgt durch RP-HPLC an C8 mit einem ansteigenden Gradienten von TFA in Acetonitril.
Ausbeute: 133 mg (60 %).

### Beispiel 40

### Festphasensynthese von H-(III T)₂-Ala-OH

125 mg (0,1 mmol) tert.-Butyloxycarbonyl-alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 200 mg (0,5 mmol) IIIBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25 minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 29 mg (45 %).

### Beispiel 41

### Festphasensynthese von H-(III T)₈-Ala-OH

125 mg (0,1 mmol) tert.-Butyloxycarbonyl-alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 200 mg (0,5 mmol) IIIBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25 minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 47 mg (20 %).

### Beispiel 42

### Festphasensynthese von H-(III T)₁₂-Ala-OH

164 mg (0,1 mmol) tert.-Butyloxycarbonyl-2-chlorbenzyloxycarbonyl-Lysin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 200 mg (0,5 mmol) IIIBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25 minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 174 mg (50 %).

### Beispiel 43

### Festphasensynthese von H-(III C-III T)-Ala-OH

125 mg (0,1 mmol) tert.-Butyloxycarbonyl-alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 243 mg (0,5 mmol) IIIBocC^{Bz} und 200 mg (0,5 mmol) IIIBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25 minütige Behandlung mit einer Lösung von 200 µl Trifluormethansulfonsäure in 2 ml Trifluoressigsäure. Die Abspaltung der Benzoyl-Schutzgruppe erfolgt durch Einwirkung von konzentrierter Ammoniak-Lösung bei 55°C. Die Reindarstellung erfolgt durch RP-HPLC an C8 mit einem ansteigenden Gradienten von TFA in Acetonitril.
Ausbeute: 28 mg (45 %).

### Beispiel 44

### Untersuchung der DNA-Einzelstrangbindung durch Gel-Shift-Analysen

1 µg Oligonukleotid von entsprechender Basensequenz wird mit Polynukleotidkinase und γ-ATP am 5'-Ende in gängiger Weise in einem Volumen von 10 µl markiert (Sambrook, Fritsch, Maniatis: Molecular Cloning, A Laboratory Manual, Cold Spring Harbor, 1989). Nach der Markierung wird die Probe zur Denaturierung des Enzyms bei 70°C für 10 Min. erhitzt und anschließend mit 9 µg nicht markiertem Oligomer vermischt. 1 µl dieses Ansatzes wird mit einer gewünschten Menge zu testendem Nukleinsäure-bindenden Oligomer versetzt (1-10 µg) und in einem Volumen von 20 µl 30 Min bei 22°C (Raumtemperatur) inkubiert (Hybridisierung). Danach wird die Probe 30 min, auf Eis gestellt. Ein nicht hybridisiertes markiertes Oligomer wird in gleicher Weise behandelt und dient als Kontrolle. Die Proben werden auf ein 15 % Polyacrylamidgel mit 1x Tris-Borat-EDTA-Puffer aufgetragen. Das Gel und der Puffer wurden im Kühlschrank (8°C) vorgekühlt, die Elektrophorese wurde über Nacht mit 55 V im Kühlschrank laufen gelassen. Nach der Elektrophorese wurde ein Autoradiogram auf Agfa-Film erstellt (Exponierzeiten 1-16 Stunden).

### Beispielversuch 45

### Nachweis der Strangverdrängung in doppelsträngiger Plasmid-DNA durch Nukleinsäure-bindende Oligomere

Die Tests wurden wie folgt durchgeführt:

(Die im Beispiel eingesetzte Plasmid-DNA ist nur ein Modell-Substrat im Test. Andere Plasmide, die Poly-Adenin Sequenzbereiche mit definierten Abständen zueinander enthalten, sind ebenfalls verwendbar).

Doppelsträngige, zirkuläre Plasmid-DNA von 4880 Basenpaaren Länge, die zwei Poly-Adenin Sequenzbereiche mit mindestens neun aufeinander folgenden Adeninnukleotiden im Abstand von 1150 Basenpaaren enthält, wird in den hier beschriebenen Tests verwendet.

Sechs parallel angesetzte Proben, bezeichnet (1-6), enthielten je 1,0 µg ungeschnittene Plasmid-DNA in 14 µl H₂O. Den Proben 3 bis 6 wurden je 1 µl Lösung von 0,01 µg, 0,1 µg, 1,0 µg und 2,0 µg Nukleinsäuren-bindenden Oligomeren aus Beispiel 25 H-(IIIT)₁₂-Lys-OH zugesetzt und in geschlossenen Eppendorfreaktionsgefäßen 45 min bei 37°C inkubiert. Anschließend wurde in alle Proben 4 µl Puffer (250 mM Na-Acetat, 1 M NaCl, 2,5 % Glycerin, 5 mM ZnCl₂, pH 4,4) und in die Proben 2 bis 6 je 1 µl S1-Nuclease aus Aspergillus oryzae, (Fa. Boehringer Mannheim) mit einer Aktivität von 10 U/µl gegeben. Nach einer Inkubation von 15 Minuten bei 30°C wurden die Proben auf Eis gesetzt, 1 µl 0,5 M EDTA und 3 µl Auftragspuffer (50% Glycerin, 0,25 % Bromphenolblau in 40 mM Tris-HCl, 20 mM Natriumacetat, 1 mM EDTA, pH = 7,2) zugegeben und ohne Zeitverzug die Proben über 1,2 %-Agarosegele elektrophoretisch aufgetrennt und nach Anfärbung mit Ethidiumbromid die Größe der entstandenden Plasmid-Fragmente im Gel im Vergleich zu einem Molekulargewichtsstandard (1-kb Leiter, Fa. Gibco-BRL, D-7514 Eggenstein) auf dem Transilluminator bei 254 nm UV-Licht bestimmt.

Es zeigte sich, daß in den Proben mit einer Konzentration > 0,1 µg Oligomere aus Beispiel 25 (Proben 5 und 6) DNA-Fragmente von 4880 Basenpaaren (Plasmid-Linearisierung), und 3720 sowie 1150 Basenpaaren (sequenzselektive Fragmentierung) sichtbar waren.

Mit einem modifizierten Testansatz bei dem in die Proben anstelle der zirkulären, ungeschnittenen Plasmid-DNA eine Plasmid-DNA gegeben wurde, die durch Restriktionsendonukleaseverdau in unmittelbarer Nachbarschaft von einer der beiden Poly-Adenin Sequenzbereiche linearisiert war, waren ebenfalls in Proben 5 und 6 die DNA-Fragmente von 3730 und 1150 Basenpaaren Länge nachweisbar.

Mit diesen Testreihen war die konzentrationsabhängige und sequenzselektive Bindung des Oligomers aus Beispiel 25 an doppelsträngige DNA und der Nachweis der dadurch entstehenden Einzelstrang-DNA durch S1-Nukleaseverdau bei hohen Salzkonzentrationen (einzelstrangspezifische Aktivität von S1-Nuklease) nachweisbar.

### Beispiel 46

### Inhibition der Genexpression (in-vitro-Translations-Test)

Zur in-vitro Translation wurde ein Kaninchenretikulozytenlysat der Fa. Promega, Madison, Wisconsin verwendet, sowie in-vitro transkribierte mRNA des tat-Gens aus HIV-I und der Delta-Untereinheit des Acetylcholinrezeptors aus Torpedo californica. Andere Gene können in gleicher Weise verwendet werden. Die cDNA-Konstrukte der Gene wurde mit SP6- bzw. T7-RNA-Polymerase in gängiger Weise transkribiert (Sambrook et al., dito), das DNA-Plasmid wurde anschließend durch DNase verdaut, die mRNA phenolisiert und dreimal mit Ethanol gefällt. 1-2 µg der erhaltenen mRNA wurde in Gegenwart von ³⁵S-markiertem Cystein zur in-vitro Translation eingesetzt. Die Analyse des gebildeten radioaktiven Proteins erfolgte auf einem 6-18 % bzw 6-10 % diskontinuierlichen SDS-AGE nach Laemmli, U.K. (1970) Nature 227, 683-685.

Zur quantitativen Messung der Translationsinhibition durch Nukleinsäure-bindende Oligomere wurde zur mRNA eine gewünschte Menge Oligomer (0,01-2 µg) hinzugegeben und dann wie oben beschrieben in Kaninchenretikulozytenlysat translatiert. Autoradiographien von SDS-Polyacrylamidelektrophoresegelen der Testansätze wurden mit einem Scanner quantitativ ausgewertet.

### Beispiel 47

### Stabilität gegenüber Proteasen

Zur Untersuchung der Proteasestabilität am Beispiel der Nukleinsäure-bindenden Oligomere aus Beispiel 24 und 25 wurden Ansätze von jeweils 75 µg Oligomer mit 5 ml Protease, wie in der nachfolgenden Tabelle aufgeführt, und 5 µl Proteasepuffer (1 M Tris/HCl; pH 7,5; 0,4 M CaCl₂) auf 50 µl Gesamtvolumen mit bidest. H₂O aufgefüllt und 3 Stunden bei 37°C inkubiert.

Neben den gereinigten, definierten Proteasen wurden aus Zellextrakte vom T-Lymphozyten und Blut für die Stabilitätsuntersuchungen eingesetzt. 50 ml Zellsuspension wurden hierzu bei 2 000 UpM abzentrifugiert und in 500 µl Tris/HCl Puffer pH 7,5; 40 mM CaCl₂ aufgenommen und mit 5 µl 20 %igem SDS lysiert. Nach Dialyse gegen 0,1 M Tris/HCl pH 7,5; 40 mM CaCl₂ und Pufferwechsel nach 4 Stunden wurden 5 µl direkt im Test eingesetzt. Blut wurde direkt mit H₂O bidest. lysiert und dann 5 µl im Test eingesetzt. Als Positivkontrollen für den Verdau durch Proteasen wurden Rinderserumalbumin (BSA) und Lysozym verwendet.

Zu den Ansätzen wurde das gleiche Volumen Proteinauftragspuffer (8 % β-Mercaptoethanol, 3,5 % SDS, 8 M Harnstoff, 125 mM Tris/HCL pH 8; 0,02 % Bromphenolblau, 20 % Glycerin) zugegeben.

30 µl der Ansätze wurden dann auf ein Polyacrylamidgel (Sammelgel 4 %ig, Trenngel 15 %ig) aufgetragen und 2,5 Stunden bei 280 V/40 mA elektrophoretisiert.

Anschließend wurde das Gel über UV-Shadowing analysiert. Dazu wurde das Gel auf eine Merck DC Fertigplatte (Kieselgel 60 F 254) mit UV-Indikator gelegt und bei 254 nm ausgewertet.

Darüber hinaus wurde eine Silberfärbung der Proteinbanden durchgeführt. Dazu wurde das Gel in folgenden Lösungen inkubiert:
1. 50 % Methanol, 5 % TCA 30 Minuten Inkubation
2. 50 % Methanol, 5 % TCA, 1 % CuCl₂, ZnCl₂ 15 Minuten
3. 10 % Ethanol, 5 % Essigsäure 15 Minuten
3. 0,01 % KMnO₄, in Wasser 15 Minuten
4. 10 % Ethanol, 5 % Essigsäure 10 Minuten 2
5. 10 % Ethanol 15 Minuten
7. H₂O 15 Minuten
8. 0,01 % AgNO₃ 10 Minuten
9. H₂O 20 Sekunden
10. 10 % K₂CO₃ 1 Minute
11. Entwickler (0,0075 % Formaldehyd, 10 % H₂CO₃) 6-10 Minuten
12. 5 % Essigsäure, 10 % Ethanol, 1 Minute
13. H₂O 2 Stunden

Die Gele wurden entweder in 5 % Glycerin in Plastikfolien aufbewahrt oder in einem Geltrockner behandelt.

Die Ergebnisse sind in der nachstehenden Tabelle dargestellt. Die Nukleinsäure-bindenden Oligomere aus Beispiel 24 und 25 sind gegen die getesteten Proteasen und Zellextrakte stabil.

| **Proteasestabilität von Nukleinsäure-bindenden Oligomeren aus Beispiel 24 und 25** | | |
|---|---|---|
| **Protease** | **H-(IIIT)**_{**8**}**-Ala-OH** | **H**-**(IIIT)**_{**12**}**-Lys-OH** |
| **Proteinase K** | **+** | **+** |
| **Pronase E** | **+** | **+** |
| **Trypsin** | **+** | **+** |
| **Endoprotease LYS-C** | **+** | **+** |
| **V8 Protease (GLU-C)** | **+** | **+** |
| **Protease XXI** | **+** | **+** |
| **Protease IX** | **+** | **+** |

| **Extrakte von:** | | |
|---|---|---|
| **T-Lymphozyten Zellen (HUT, U 937)** | **+** | **+** |
| **Blut** | **+** | **+** |

### Beispiel 48

### Stabilität gegenüber Nukleasen

Zur Untersuchung der Nukleasestabilität von Nukleinsäure-bindenden Oligomeren aus Beispiel 24 und 25 wurden Ansätze von jeweils 75 µg Oligomer mit 5 µl Nuklease (40 Units), wie in der Tabelle aufgeführt, 5 µl Nukleasepuffer (S1 Nuklease-Puffer 0,3 M Kaliumacetat; pH 4,6; 2,5 M NaCl, 10 mM ZnSO₄, 50 % Glycerin) auf 50 µl Gesamtvolumen mit bidest. H₂O aufgefüllt und 3 Stunden bei 37°C inkubiert.

Neben den gereinigten, definierten Nukleasen wurden auch Zellextrakte vom T-Lymphozyten und Blut für die Stabilitätsuntersuchungen eingesetzt. 50 ml Zellsuspension wurden hierzu bei 2 000 UpM abzentrifugiert und in 500 µl Tris/HCl Puffer pH 7,5; 40 mM CaCl₂ aufgenommen und mit 5 µl 20 %igem SDS lysiert. Nach Dialyse gegen 0,1 M Tris/HCl pH 7,5; 40 mM CaCl₂ und Pufferwechsel nach 4 Stunden wurden 5 µl direkt im Test eingesetzt. Blut wurde direkt nach Lyse mit H₂O bidest. eingesetzt. Als Positivkontrollen für den Verdau durch Nukleasen wurde ein 25mer Oligonukleotiddiester eingesetzt.

Zu den Ansätzen wurde das gleiche Volumen Proteinauftragspuffer (8 % β-Mercaptoethanol, 3,5 % SDS, 8 M Harnstoff, 125 mM Tris/HCL pH 8; 0,02 % Bromphenolblau, 20 % Glycerin) hinzugefügt.

30 µl der Ansätze wurden dann auf ein Polyacrylamidgel (Sammelgel 4 %ig, Trenngel 15 %ig) aufgetragen und 2,5 Stunden bei 280 V/40 mA elektrophoretisiert.

Anschließend wurde das Gel über UV-Shadowing analysiert. Dazu wurde das Gel auf eine Merck DC Fertigplatte (Kieselgel 60 F 254) mit UV-Indikator gelegt und bei 254 nm ausgewertet. Die Ergebnisse sind in der nachfolgenden Tabelle dargestellt. Die Oligomere sind gegenüber den getesteten Nukleasen und Zellextrakten stabil.

| **Nukleasestabilität von Nukleinsäure-bindenden Oligomeren aus Beispiel 24 und 25** | | |
|---|---|---|
| **Nuklease** | **H-(IIIT)**_{**8**}**-Ala-OH** | **H-(IIIT)**_{**12**}**-Lys-OH** |
| **S1-Nuklease** | **+** | **+** |
| **Ba131 Nuklease** | **+** | **+** |
| **T-Lymphozyten-Extrakt** | **+** | **+** |
| **Blut** | **+** | **+** |
| | | |
| **("+" bedeutet Stabilität kein enzymatischer Abbau)** | | |

## Patentansprüche

1. Verbindungen der allgemeinen Formel (I), 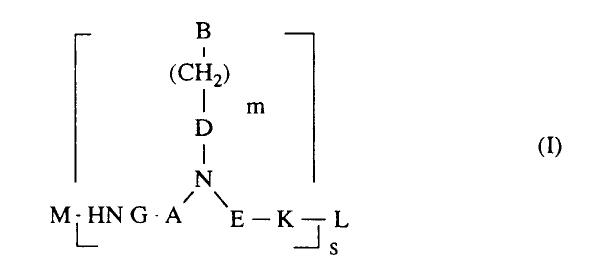 in der
A für -(CH₂)ₙ- oder -CO- steht,
B für alle natürlichen oder unnatürlichen Nukleobasen, wie z.B. Thymin, Uracil, Cytosin, Adenin, Guanin, Hypoxanthin, oder durch chemische Modifikation von diesen abgeleitete Derivate oder halogenierte Vorstufen von diesen, gegebenenfalls an den Aminogruppen mit Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert oder mit freien Aminogruppen steht,
D -(CO)ₚ- steht,
E und G unabhängig voneinander für -CHR- stehen, wobei
R Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Ornithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-a-aminobuttersäure, anderen unnatürlichen Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Iodphenylalanin, 4-Methoxyphenylalanin, 1-Triazolylalanin, 2-Pyridylalanin, 3-Pyridylalanin, 4-Pyridylalanin, 1-Naphthyalanin oder 2-Naphthylalanin, gegebenenfalls mit Schutzgruppen, in ihrer D- oder L-Form oder gegebenenenfalls
E und G über eine Alkylkette -(CH₂)_{q} miteinander verknüpft sind,
K für -CO-, -SO₂- oder -CH₂- steht,
L ein Carrier-System, Reporter-Ligand, eine löslichkeitsvermittelnde Gruppe oder Wasserstoff sein kann,
M unabhängig von L ein Carrier-System, Reporter-Ligand, eine löslichkeitsvermittelnde Gruppe oder Wasserstoff sein kann,
m 0, 1, 2 oder 3 sein kann,
n 0, 1, 2, 3 oder 4 sein kann,
p 0, 1 oder 2 sein kann,
q 0, 1 oder 2 sein kann, und
s Werte zwischen 1 und 30 annehmen kann.

2. Verbindungen der allgemeinen Formel (I), gemäß Anspruch 1
in der
A für -(CH₂)ₙ- oder -CO- steht,
B für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
D für -(CO)ₚ- steht,
E und G unabhängig voneinander für -CHR- stehen, wobei
R Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Omithin, Asparagin, Asparaginsäure, Glutamin, Glutaminsäure, Arginin, Prolin, Hydroxypyrolin, Sarcosin, Dehydroaminosäuren, wie z.B. Dehydroalanin, Dehydro-α-aminobuttersäure, anderen unnatürlichen Aminosäuren wie Phenylglycin, 2-Pyridylalanin, 3-Pyridylalanin, 4-Pyridylalanin, 1-Naphthyalanin oder 2-Naphthylalanin, gegebenenfalls mit Schutzgruppen, in ihrer D- oder L-Form oder gegebenenenfalls
E und G über eine Alkylkette -(CH₂)_{q} miteinander verknüpft sind,
K -CO-, -SO₂- oder -CH₂- sein kann,
L ein Carrier-System, Reporter-Ligand, eine löslichkeitsvermittelnde Gruppe oder Wasserstoff sein kann,
M unabhängig von L ein Carrier-System, Reporter-Ligand, eine löslichkeitsvermittelnde Gruppe oder Wasserstoff sein kann,
m 0, 1, 2 oder 3 sein kann,
n 0, 1, 2 oder 3 sein kann,
p 0 oder 1 sein kann,
q 0, 1 oder 2 sein kann, und
s Werte zwischen 3 und 20 annehmen kann.

3. Verbindungen der allgemeinen Formel (I), gemäß Anspruch 1
in der
A für -(CH₂)ₙ- oder -CO- steht,
B für alle natürlichen Nukleobasen, wie z.B. Thymin, Uracil, Cytosin, Adenin, Guanin oder Hypoxanthin oder halogenierte Vorstufen von diesen, gegebenenfalls an den Aminogruppen substituiert durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen oder mit freier Aminogruppe steht,
D für -(CO)ₚ- steht,
E und G unabhängig voneinander für -CHR- stehen, wobei
R Für H oder einen Rest einer natürlichen oder unnatürlichen Aminosäure steht, z.B. aus Glycin, Alanin, Valin, Leucin, Isoleucin, Serin, Threonin, Cystein, Methionin, Phenylalanin, Thyrosin, Histidin, Tryptophan, Lysin, Omithin, Asparagin, Asparaginsäure, Glutamin, Glutaminsäure, Arginin, Prolin, Hydroxypyrolin, Sarcosin oder Dehydroaminosäuren, wie z.B. Dehydroalanin, Dehydro-α-aminobuttersäure, gegebenenfalls mit Schutzgruppen, in ihrer D- oder L-Form oder gegebenenenfalls
E und G über eine Alkylkette -(CH₂)_{q} miteinander verknüpft sind,
K -CO- ist,
L ein Carrier-System, Reporter-Ligand, eine löslichkeitsvermittelnde Gruppe oder Wasserstoff sein kann,
M unabhängig von L ein Carrier-System, Reporter-Ligand, eine löslichkeitsvermittelnde Gruppe oder Wasserstoff sein kann,
m 0, 1 oder 2 sein kann,
n 0, 1, 2 oder 3 sein kann,
p 0 oder 1 sein kann,
q 0 oder 1 sein kann und
s Werte zwischen 3 und 18 annehmen.

4. Arzneimittel enthaltend eine oder mehrere Verbindungen aus den Ansprüchen 1 bis 3.

5. Verwendung von Verbindungen gemäß den Ansprüchen 1 bis 3 zur Herstellung von Arzneimitteln.

## Claims

1. Compounds of the general formula (I), 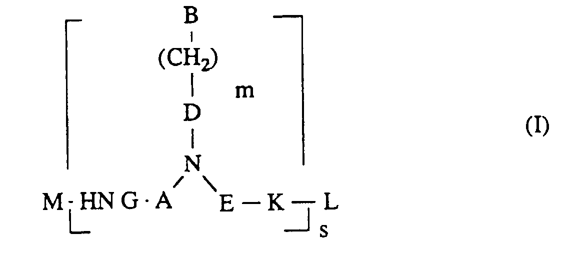 in which
A represents -(CH₂)ₙ- or -CO-,
B represents all natural or unnatural nucleobases, such as, for example, thymine, uracil, cytosine, adenine, guanine or hypoxanthine, or derivatives derived therefrom by chemical modification, or halogenated precursors thereof, optionally substituted on the amino groups by protective groups such as acetyl, trifluoroacetyl, trichloroacetyl, benzoyl, phenylacetyl, benzyloxycarbonyl, tert-butyloxycarbonyl, allyloxycarbonyl or (9-fluorenyl)methoxycarbonyl, or other protective groups which are customary in peptide and nucleic acid chemistry, or possessing free amino groups,
D represents -(CO)ₚ-,
E and G, independently of each other, represent -CHR-, where
R represents H or a residue of a natural or unnatural amino acid, e.g. from glycine, alanine, valine, leucine, isoleucine, serine, threonine, cysteine, methionine, phenylalanine, tyrosine, histidine, tryptophan, lysine, ornithine, asparagine, aspartic acid, glutamine, glutamic acid, arginine, proline, hydroxyproline or sarcosine, dehydroamino acids, such as, for example, dehydroalanine or dehydro-α-aminobutyric acid, other unnatural amino acids, such as phenylglycine, 4-nitrophenylalanine, 3-nitrophenylalanine, 2-nitrophenylalanine, 2-, 3 - or 4-aminophenylalanine, 3,4-dichlorophenylalanine, 4-iodophenylalanine, 4-methoxyphenylalanine, 1-triazolylalanine, 2-pyridylalanine, 3-pyridylalanine, 4-pyridylalanine, 1-naphthylalanine or 2-naphthylalanine, optionally possessing protective groups, in their D or L form, or, optionally,
E and G are linked together via an alkyl chain -(CH₂)_{q}-,
K represents -CO-, -SO₂- or -CH₂-,
L can be a carrier system, reporter ligand, a solubility-mediating group or hydrogen,
M can, independently of L, be a carrier system, reporter ligand, a solubility-mediating group or hydrogen,
m can be 0, 1, 2 or 3,
n can be 0, 1, 2, 3 or 4,
p can be 0, 1 or 2,
q can be 0, 1 or 2, and
s can assume values of between 1 and 30.

2. Compounds of the general formula (I) according to Claim 1
in which
A represents -(CH₂)ₙ- or -CO-,
B represents all natural nucleobases, such as, for example, thymine, uracil, cytosine, adenine, guanine or hypoxanthine, or halogenated precursors thereof, optionally substituted on the amino groups by protective groups such as acetyl, trifluoroacetyl, trichloroacetyl, benzoyl, phenylacetyl, benzyloxycarbonyl, tert-butyloxycarbonyl, allyloxycarbonyl or (9-fluorenyl)methoxycarbonyl, or other protective groups which are customary in peptide and nucleic acid chemistry, or possessing free amino groups,
D represents -(CO)ₚ-,
E and G, independently of each other, represent -CHR-, where
R represents H or a residue of a natural or unnatural amino acid, e.g. from glycine, alanine, valine, leucine, isoleucine, serine, threonine, cysteine, methionine, phenylalanine, tyrosine, histidine, tryptophan, lysine, ornithine, asparagine, aspartic acid, glutamine, glutamic acid, arginine, proline, hydroxyproline or sarcosine, dehydroamino acids, such as, for example, dehydroalanine or dehydro-α-aminobutyric acid, other unnatural amino acids, such as phenylglycine, 2-pyridylalanine, 3-pyridylalanine, 4-pyridylalanine, 1-naphthylalanine or 2-naphthylalanine, optionally possessing protective groups, in their D or L form, or, optionally,
E and G are linked together via an alkyl chain -(CH₂)_{q}-,
K can be -CO-, -SO₂- or -CH₂-,
L can be a carrier system, reporter ligand, a solubility-mediating group or hydrogen,
M can, independently of L, be a carrier system, reporter ligand, a solubility-mediating group or hydrogen,
m can be 0, 1, 2 or 3,
n can be 0, 1, 2 or 3,
p can be 0 or 1,
q can be 0, 1 or 2, and
s can assume values of between 3 and 20.

3. Compounds of the general formula (I) according to Claim 1
in which
A represents -(CH₂)ₙ- or -CO-,
B represents all natural nucleobases, such as, for example, thymine, uracil, cytosine, adenine, guanine or hypoxanthine, or halogenated precursors thereof, optionally substituted on the amino groups by protective groups such as acetyl, trifluoroacetyl, trichloroacetyl, benzoyl, phenylacetyl, benzyloxycarbonyl, tert-butyloxycarbonyl, allyloxycarbonyl or (9-fluorenyl)methoxycarbonyl, or other protective groups which are customary in peptide and nucleic acid chemistry, or possessing free amino groups,
D represents -(CO)ₚ-,
E and G, independently of each other, represent -CHR-, where
R represents H or a residue of a natural or unnatural amino acid, for example from glycine, alanine, valine, leucine, isoleucine, serine, threonine, cysteine, methionine, phenylalanine, tyrosine, histidine, tryptophan, lysine, ornithine, asparagine, aspartic acid, glutamine, glutamic acid, arginine, proline, hydroxyproline or sarcosine, or dehydroamino acids, such as, for example, dehydroalanine or dehydro-α-aminobutyric acid, optionally possessing protective groups, in their D or L form, or, optionally,
E and G are linked together via an alkyl chain -(CH₂)_{q}-,
K is -CO-,
L can be a carrier system, reporter ligand, a solubility-mediating group or hydrogen,
M can, independently of L, be a carrier system, reporter ligand, a solubility-mediating group or hydrogen,
m can be 0, 1 or 2,
n can be 0, 1, 2 or 3,
p can be 0 or 1,
q can be 0 or 1, and
s can assume values of between 3 and 18.

4. Medicament containing one or more compounds from the Claims 1 to 3.

5. Use of compounds according to Claims 1 to 3 for preparing medicaments.

## Revendications

1. Composés de formule générale (I), 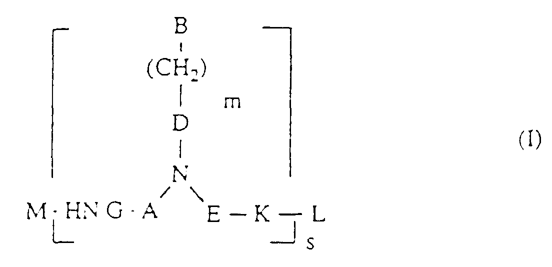 dans laquelle
A représente -(CH₂)ₙ- ou -CO-,
B représente toutes les bases nucléiques naturelles ou non naturelles, comme par exemple la thymine, l'uracile, la cytosine, l'adénine, la guanine, l'hypoxanthine, ou des dérivés obtenus à partir de ces bases par modification chimique, ou des précurseurs halogénés de ces bases, éventuellement substitués sur les groupes amino avec des groupes de protection tels que l'acétyle, le trifluoroacétyle, le trichloroacétyle, le benzoyle, le phénylacétyle, le benzyloxycarbonyle, le tert.-butyloxycarbonyle, un allyloxycarbonyle, le (9-fluorényl)méthoxycarbonyle ou d'autres groupes de protection habituels dans la chimie des peptides et des acides nucléiques, ou contenant des groupes amino libres,
D représente -(CO)ₚ-,
E et G représentent -CHR- indépendamment l'un de l'autre, et
R représente H ou un résidu d'un aminoacide, naturel ou non naturel, par exemple la glycine, l'alanine, la valine, la leucine, l'isoleucine, la sérine, la thréonine, la cystéine, la méthionine, la phénylalanine, la thyrosine, l'histidine, le tryptophane, la lysine, l'ornithine, l'asparagine, l'acide asparagique, la glutamine, l'acide glutamique, l'arginine, la proline, l'hydroxypyrroline, la sarcosine, de déhydroaminoacides, comme par exemple la déhydroalanine, l'acide déhydro-α-aminobutyrique, d'autres aminoacides non naturels tels que la phénylglycine, la 4-nitrophénylalanine, la 3-nitrophénylalanine, la 2-nitrophénylalanine, la 2-, la 3-ou la 4-aminophénylalanine, la 3,4-dichlorophénylalanine, la 4-iodophénylalanine, la 4-méthoxyphénylalanine, la 1-triazolylalanine, la 2-pyridylalanine, la 3-pyridylalanine, la 4-pyridylalanine, la 1-naphtylalanine ou la 2-naphtylalanine, éventuellement avec des groupes de protection, dans leur forme D ou L, ou éventuellement
E et G sont couplés l'un à l'autre par l'intermédiaire d'une chaîne alkyle -(CH₂)ₙ-
K représente -CO-, -SO₂- ou -CH₂-,
L peut être un système porteur, un ligand messager, un groupe conférant une solubilité ou l'hydrogène,
M peut être, indépendamment de L, un système porteur, un ligand messager, un groupe conférant une solubilité ou l'hydrogène,
m peut valoir 0, 1, 2 ou 3,
n peut valoir 0, 1, 2, 3 ou 4,
p peut valoir 0, 1 ou 2,
q peut valoir 0, 1 ou 2 et
s peut prendre des valeurs comprises entre 1 et 30.

2. Composés de formule générale (I) selon la revendication 1, dans lesquels
A représente -(CH₂)ₙ- ou -CO-,
B représente toutes les bases nucléiques naturelles, comme par exemple la thymine, l'uracile, la cytosine, l'adénine, la guanine, l'hypoxanthine, ou des précurseurs halogénés de ces bases, éventuellement substitués sur les groupes amino avec des groupes de protection tels que l'acétyle, le trifluoroacétyle, le trichloroacétyle, le benzoyle, le phénylacétyle, le benzyloxycarbonyle, le tert.-butyloxycarbonyle, un allyloxycarbonyle, le (9-fluorényl)méthoxy-carbonyle ou d'autres groupes de protection habituels dans la chimie des peptides et des acides nucléiques, ou contenant des groupes amino libres,
D représente -(CO)ₚ-,
E et G représentent -CHR- indépendamment l'un de l'autre, et
R représente H ou un résidu d'un aminoacide, naturel ou non naturel, par exemple la glycine, l'alanine, la valine, la leucine, l'isoleucine, la sérine, la thréonine, la cystéine, la méthionine, la phénylalanine, la thyrosine, l'histidine, le tryptophane, la lysine, l'ornithine, l'asparagine, l'acide asparagique, la glutamine, l'acide glutamique, l'arginine, la proline, l'hydroxypyrroline, la sarcosine, de déhydroaminoacides, comme par exemple la déhydroalanine, l'acide déhydro-α-aminobutyrique, d'autres aminoacides non naturels tels que la phénylglycine, la 2-pyridylalanine, la 3-pyridylalanine, la 4-pyridylalanine, la 1-naphtylalanine ou la 2-naphtylalanine, éventuellement avec des groupes de protection, dans leur forme D ou L, ou éventuellement
E et G sont couplés l'un à l'autre par l'intermédiaire d'une chaîne alkyle -(CH₂)_{q}-
K représente -CO-, -SO₂- ou -CH₂-,
L peut être un système porteur, un ligand messager, un groupe conférant une solubilité ou l'hydrogène,
M peut être, indépendamment de L, un système porteur, un ligand messager, un groupe conférant une solubilité ou l'hydrogène,
m peut valoir 0, 1, 2 ou 3,
n peut valoir 0, 1, 2 ou 3,
p peut valoir 0 ou 1,
q peut valoir 0, 1 ou 2 et
s peut prendre des valeurs comprises entre 3 et 20.

3. Composés de formule générale (I) selon la revendication 1, dans lesquels
A représente -(CH₂)ₙ- ou -CO-,
B représente toutes les bases nucléiques naturelles, comme par exemple la thymine, l'uracile, la cytosine, l'adénine, la guanine, l'hypoxanthine, ou des précurseurs halogénés de ces bases, éventuellement substitués sur les groupes amino avec des groupes de protection tels que l'acétyle, le trifluoroacétyle, le trichloroacétyle, le benzoyle, le phénylacétyle, le benzyloxycarbonyle, le tert.-butyloxycarbonyle, un allyloxycarbonyle, le (9-fluorényl)méthoxy-carbonyle ou d'autres groupes de protection habituels dans la chimie des peptides et des acides nucléiques, ou contenant des groupes amino libres,
D représente -(CO)ₚ-,
E et G représentent -CHR- indépendamment l'un de l'autre, et
R représente H ou un résidu d'un aminoacide, naturel ou non naturel, par exemple la glycine, l'alanine, la valine, la leucine, l'isoleucine, la sérine, la thréonine, la cystéine, la méthionine, la phénylalanine, la thyrosine, l'histidine, le tryptophane, la lysine, l'ornithine, l'asparagine, l'acide asparagique, la glutamine, l'acide glutamique, l'arginine, la proline, l'hydroxypyrroline, la sarcosine, de déhydroaminoacides comme par exemple la déhydroalanine, l'acide déhydro-α-aminobutyrique, éventuellement avec des groupes de protection, dans leur forme D ou L, ou éventuellement
E et G sont couplés l'un à l'autre par l'intermédiaire d'une chaîne alkyle -(CH₂)_{q}-
K représente -CO-,
L peut être un système porteur, un ligand messager, un groupe conférant une solubilité ou l'hydrogène,
M peut être, indépendamment de L, un système porteur, un ligand messager, un groupe conférant une solubilité ou l'hydrogène,
m peut valoir 0, 1 ou 2,
n peut valoir 0, 1, 2 ou 3,
p peut valoir 0 ou 1,
q peut valoir 0 ou 1 et
s peut prendre des valeurs comprises entre 3 et 18.

4. Médicament contenant un ou plusieurs des composés selon l'une des revendications 1 à 3.

5. Utilisation de composés selon les revendications 1 à 3 pour la préparation de médicaments.
